# EUROPÄISCHE PATENTSCHRIFT

(11) **EP 1 198 488 B1**
(45) Veröffentlichungstag und Bekanntmachung des Hinweises auf die Patenterteilung: **14.09.2005**
(21) Anmeldenummer: 00954430.5
(22) Anmeldetag: 05.07.2000
(51) Int. Cl.: C08G 63/664, C08G 81/00, A61L 27/18, A61K 47/48

(54) **BIOABBAUBARE BLOCKCOPOLYMERE MIT MODIFIZIERBARER OBERFLÄCHE**
BIODEGRADABLE BLOCK COPOLYMERS WITH MODIFIABLE SURFACE
COPOLYMERES EN BLOC BIODEGRADABLES AVEC SURFACE MODIFIABLE

(30) Priorität: 05.07.1999 DE 19930729
(43) Veröffentlichungstag der Anmeldung: 24.04.2002
(73) Patentinhaber: Göpferich, Achim, Prof. Dr., 93161 Sinzing (DE)
(72) Erfinder: GÖPFERICH, Achim, Prof. Dr., D-93161 Sinzing (DE); TESSMAR, Jörg, D-93051 Regensburg (DE); SCHULZ, Michaela, D-93051 Regensburg (DE); BLUNK, Torsten, D-93080 Pentling (DE); MIKOS, Antonios, Houston, TX 77098 (US)
(74) Vertreter: Ahrens, Gabriele
(86) Internationale Anmeldenummer: PCT/EP2000/006313
(87) Internationale Veröffentlichungsnummer: WO 2001/002460

(56) Entgegenhaltungen:
- EP-A- 0 844 269
- WO-A-95/03356

## Beschreibung

Gegenstand der Erfindung sind Diblockcopolymere mit einem hydrophoben bioabbaubaren Anteil und einem hydrophilen biokompatiblen Anteil, die die selektive Anbindung von oberflächenmodifizierenden Substanzen erlauben und zugleich die unselektive Adhäsion unerwünschter Substanzen unterdrücken können, und daraus hergestellte Formkörper.
Derartige Diblockcopolymere eignen sich insbesondere als Träger für Zellen für die Gewebezüchtung, als Träger für Wirkstoffe wie Medikamente, insbesondere zur kontrollierten Freigabe (drug delivery system) und zur gezielten Verabreichung von Wirkstoffen (drug targeting).

Biomaterialien, zu denen die erfindungsgemäßen Diblockcopolymere gehören, spielen bei einer Reihe medizinischer Applikationen eine herausragende Rolle. Unter Biomaterialien versteht man Substanzen, die im menschlichen oder tierischen Körper als Ersatzstoffe für körpereigene Materialien eine bestimmte Funktion übernehmen. Beispiele hierfür sind Metalle oder Polymere, wie man sie zum Beispiel bei Totalendoprothesen im Bereich des Hüftgelenks verwendet. Ein Nachteil vieler Biomaterialien, die nur vorübergehend im Körper eingesetzt werden, wie zum Beispiel Nägel oder Platten im Bereich der Chirurgie besteht darin, dass sie nach der Applikation entfernt werden müssen. Aus diesem Grunde setzte zu Beginn der 70er Jahre die intensive Suche nach bioabbaubaren Materialien ein, die sich während der Applikation in körperverträgliche Bruchstücke abbauen.
Unter 'bioabbaubar' versteht man, dass das biologische System, in welches man ein Material bringt, zu dessen Abbau beiträgt [Vert, M. et al. "Degradable Polymers and Plastics" Redwood Press Ltd. (1992) 73-92]. Besonders hervorzuheben sind bioabbaubare Polymermaterialien, die in Oligomere oder Monomere zerfallen. Als Beispiele für deren Anwendung seien chirurgisches Nahtmaterial oder abbaubare Arzneistoffträger genannt.

Die erfolgreiche Anwendung bioabbaubarer Polymere hat zu einer intensiven Suche nach neuen synthetischen Materialien geführt, aus der eine Vielzahl verschiedener Polymerklassen resultierte, wie zum Beispiel Poly(α-hydroxyester), Poly(β-hydroxyester), Polyanhydride, Polycyanoacrylate und viele andere [Göpferich, A. (1997) 451-472;Göpferich, A. Biomaterials17 (1996a) 103-114;Göpferich, A. Eur.J.Pharm.Biopharm.42 (1996b) 1-11].

Besonderes Kennzeichen dieser Polymere ist ihre geringe Löslichkeit in wässrigen Medien, die sich erst durch den Polymerkettenabbau, d.h. die Hydrolyse zu niedermolekularen Oligomeren oder Monomeren, verbessert und damit zur Erosion dieser Materialien führt.

Neben der Entwicklung von synthetischen bioabbaubaren Polymeren setzte gleichzeitig eine intensive Suche nach natürlichen Polymeren ein, die ähnliche Eigenschaften besitzen. Beispiele hierfür sind Kollagen, Hyaluronsäure, Alginat und Zellulosederivate [Park, K. et al. Biodegradable Hydrogels for Drug Delivery (1993)]. Bei diesen Substanzen wird zum Teil in Kauf genommen, dass sie eine erhöhte Wasserlöslichkeit besitzen. Um die Wasserlöslichkeit herabzusetzen, werden natürliche Polymere oft chemisch modifiziert, zum Beispiel durch Veretherungen und Veresterungen funktioneller Gruppen in der Polymerkette oder durch Quervemetzungen einzelner Stränge. Als Beispiel sei hier die Quervemetzung von Kollagen, Gelatine oder Alginat genannt.

Verschiedene bioabbaubare Polymere unterscheiden sich vor allem durch die Geschwindigkeit von Polymerkettenabbau und Erosion. Dies ist für viele Anwendungen von Bedeutung, bei denen sich der Polymerkettenabbau über einen definierten Zeitraum erstrecken muss, wie zum Beispiel bei der Freigabe von Arzneistoffen.

Wesentlich für die medizinische Anwendung synthetischer, partialsynthetischer und natürlicher bioabbaubarer Polymere ist deren Verträglichkeit mit dem biologischen System, in welches sie eingebracht werden. Für Anwendungen im menschlichen oder tierischen Organismus dürfen einzelne Bauteile, wie zum Beispiel Oligomere oder Monomere nicht toxisch sein und die Polymere dürfen höchstens eine moderate Entzündungsreaktion im Gewebe auslösen.
Die oben genannten bioabbaubaren Polymere finden derzeit Verwendung zur kontrollierten Freigabe von Arzneistoffen (drug delivery) [Göpferich, A. Eur.J.Pharm.Biopharm.42 (1996b) 1-11] und als Träger für Zellen (tissue engineering) [Langer, R. and Vacanti, J.P. Science260 (1993) 920-926].

Im Rahmen des drug delivery geben bioabbaubare Polymere Arzneistoffe durch Diffusion, Erosion, Quellung oder osmotische Effekte kontrolliert frei.

Im Bereich des tissue engineering setzt man bioabbaubare Polymere zum Beispiel als poröse "Schwämme" ein, auf denen Zellen adhärieren, proliferieren und differenziert werden können. Während sich die Zellen zu einem Gewebeverband entwickeln, baut sich der Polymerträger ab und es resultiert ein Gewebe, welches sich in den menschlichen oder tierischen Körper transplantieren lässt.
Beispiele für Gewebe die derzeit auf diese Weise hergestellt werden, sind u.a. Knorpel, Knochen, Fettgewebe und Gefäße.

Die Anwendung bioabbaubarer Polymere im Bereich des tissue engineering und des drug delivery stellen besondere Anforderungen an diese Materialien.
Neben der bereits erwähnten Biokompatibilität der Polymere und ihrer Abbauprodukten stellen diese Anwendungen besondere Anforderungen an die Oberflächeneigenschaften der Polymere.

Zunächst seien einige Beispiele aus dem Bereich des drug delivery genannt:
1. Man beobachtet häufig eine Adsorption von Molekülen (zum Beispiel Arzneistoffe, Proteine und Peptide) an die Polymeroberflächen. Dies kann dazu führen, dass der bioabbaubare Arzneistoffträger seine Dosis nicht im gewünschten Umfang und nicht mit der gewünschten Kinetik freisetzt. Im Extremfall kann dies auch die Inaktivierung des Wirkstoffes zur Folge haben. Die Adsorption von Wirkstoffen ist daher in vielen Fällen unerwünscht und muss unterdrückt werden.
2. Die Verträglichkeit eines bioabbaubaren Polymers hängt in hohem Maße von dessen Oberflächeneigenschaften ab. So werden diese Polymere in Form von Teilchen im Mikrometer- und Nanometerbereich von Zellen des Immunsystems, wie zum Beispiel Makrophagen nach Absorption körpereigener Proteine erkannt und anschließend phagozytiert.
   Die Kontrolle der Oberflächeneigenschaften kleiner Teilchen als parenterale Arzneiformen ist daher für deren erfolgreichen Einsatz erforderlich.
3. Man versucht seit langem, bioabbaubare Nanopartikel für die gezielte Verabreichung von Substanzen an spezifische Gewebe (zum Beispiel Tumore oder zentrales Nervensystem) einzusetzen (drug targeting). Dabei stellte sich heraus, dass körpereigenen Proteine, die auf den Partikeloberflächen adsorbiert werden, dafür verantwortlich sind, wohin diese Partikel transportiert werden [Juliano, R.L. Adv.Drug Delivery Rev.2 (1988) 31-54]. Bisher ist man nur bedingt in der Lage, eine gezielte Adsorption dieser Proteine an die Partikel zu erreichen. Polymere, welche auf einfache Art und Weise die gezielte Modifikation ihrer Oberflächen erlauben, sind daher vorteilhaft.

Auch im Bereich des tissue engineering spielen die Oberflächeneigenschaften bioabbaubarer Polymere eine bedeutende Rolle :
1. Die Wechselwirkungen zwischen Zellen und Polymer entscheiden über Zellwachstum und Zelldifferenzierung. Verantwortlich für die Anheftung der Zellen an die Polymeroberflächen sind natürliche Verankerungsmechanismen der Zellen. Proteine, wie zum Beispiel Integrine, erlauben es, dass sich Zellen an spezifische Aminosäuresequenzen anheften. Die Anheftung an bioabbaubare Polymere kommt dadurch zustande, dass Proteine aus Körperflüssigkeiten oder Zellkulturmedien unspezifisch an die Polymeroberflächen adsorbieren und sich die Zellen ihrerseits wiederum an die entsprechenden Aminosäuresequenzen der Proteine anheften. Diese unspezifische Adsorption von Proteinen bewirkt, dass sich eine Vielzahl verschiedener Zellen an die Oberfläche anheftet. Dies ist vor allem dann von Nachteil, wenn auf dem bioabbaubaren Polymer eine bestimmte Zellsorte adhäriert werden soll. Die Kontrolle über die Adsorption von Proteinen und Peptiden ist daher erwünscht.
2. Die Aminosäuresequenzen, an die Zellen anbinden, sind oft für einen Zelltypus spezifisch, d.h. belegt man die Oberfläche eines Polymers mit einer zellspezifischen Sequenz, dann adhäriert bevorzugt dieser Zelltyp
3. Die Membran einer Zelle trägt eine Reihe von Rezeptoren, wobei über diese Rezeptoren Einfluss auf das Verhalten der Zelle genommen werden kann. Befinden sich daher entsprechende "Signalstoffe", wie zum Beispiel Hormone, Wachstumsfaktoren oder Zytokine auf der Oberfläche von Polymeren, an die die Rezeptoren binden können, lässt sich über diese entsprechend belegten Polymeroberflächen das Verhalten der daran über die Rezepzoren haftenden Zellen beeinflussen.

Die oben genannten Beispiele zeigen die Bedeutung der Oberflächeneigenschaften eines bioabbaubaren Polymers bzw. die Bedeutung der Möglichkeit zur selektiven Modifizierung dieser Oberfläche für die erfolgreiche Applikation des Polymers. Die Modifizierung der Oberflächeneigenschaften bioabbaubarer Polymere ist seit einigen Jahren Ziel intensiver Forschung.

Die ersten Ansätze zur Herstellung bioabbaubarer Polymere mit modifizierbaren Oberflächen gingen davon aus, in die Polymerkette von Poly(α-hydroxyestern), wie zum Beispiel Polylactid, Monomere einzubauen, wie zum Beispiel Lysin, die eine funktionelle Gruppe enthalten, an die sich Moleküle anheften lassen [Barrera, D.A. "Synthesis and Characterization of a novel Biodegradable Polymer - Poly(lactic acid-co-lysin)" 1993, Massachusetts Institute of Technology, PHD Thesis].

Ein Nachteil dieser Polymere besteht darin, dass die funktionellen Gruppen, hier Aminogruppen, in der Oberfläche nur schwer zugänglich sind. Um dies zu verbessern, wurden an diese funktionellen Gruppen Oligopeptide angeheftet, damit das Knüpfen neuer chemischer Bindungen erleichtert wird.
Von Nachteil ist, dass bei dem erhaltenen Polymer die unspezifische Adsorption von unerwünschten Proteinen und Peptiden erfolgt.

Dies führte zu neuen Entwicklungen, um ein breiter anwendbares System zu erhalten [Patel, N., Padera, R., Sanders, G. H., Cannizzaro, S. M., Davies, M. C., Langer, R., Roberts, C. J., Tendler, S. J., Williams, P. M., and Shakesheff, K. M. "Spatially controlled Tissue Engineering on Biodegradable Polymer Surfaces". 25(1), 109-110. 1998. Controlled Release Society, Inc. Proceed. Int'l. Symp. Control. Rel. Bioact. Mater. 1998]. Dabei macht man sich die Bindung von Biotin an das Protein Avidin zunutze, die sehr spezifisch ist. Man verankert Biotin auf einer Polymeroberfläche und bindet an die Substanz, mit der man die Oberfläche belegen will ebenfalls Biotin. In Gegenwart von Avidin, welches mehrere Bindungsstellen für Biotin besitzt, kommt es dann zur gezielten Anheftung der biotinylierten Verbindung an die Oberfläche.
Ein Vorteil des Verfahrens besteht darin, dass sich auf einer Polymeroberfläche Muster erzeugen lassen. Dies hat Bedeutung für Gewebe, bei denen eine strukturierte Anordnung von Zellen erforderlich ist.
Von Nachteil ist jedoch, dass man über die Verankerung von Avidin ein Protein verwendet, welches körperfremd ist und somit zu unerwünschten Reaktionen führen kann. Zusätzlich muss die zu verankernde Substanz erst biotinyliert werden, was das Verfahren kompliziert und damit die Anwendbarkeit einschränkt. Gleichzeitig ist die Oberfläche mit Avidin belegt, was für viele Anwendungen unerwünscht ist.

Andere Methoden verwenden zur Anheftung oberflächenmodifizierender Substanzen an die Oberfläche des bioabbaubaren Polymers ein weiteres Polymer.
So wird zum Beispiel Polyethylenglykol an die zu modifizierende Oberfläche angeheftet, was die entsprechende Existenz funktioneller Gruppen auf den Oberflächen voraussetzt [US-Patent 5,906,828]. Diese funktionellen Gruppen müssen bei diesen Entwicklungen zum Teil durch chemische Reaktionen erst erzeugt werden. Dies ist ein zusätzlicher Verfahrensschritt und erhöht den Aufwand für die Anwendung dieses Verfahrens in unerwünschter Weise.

In dem US-Patent 5,330,911 ist die Verankerung von speziellen Peptidsequenzen auf Keramiken, Polyhydroxyethylmethacrylat und Polyethylenterephthalat beschrieben. Das Verfahren setzt die Existenz funktioneller Gruppen voraus und ist nicht dazu geeignet, unspezifische Adsorption zu unterdrücken.

Ein weiteres Verfahren stützt sich auf Polyalkylimin als Abstandshalter ("Spacer") zwischen der Polymeroberfläche und anzuheftender Substanz [US-Patent 5,308,641]. Das Verfahren hat die gleichen Nachteile wie für US-Patent 5,330,911 beschrieben und setzt die Existenz entsprechender funktioneller Gruppen auf der Polymeroberfläche voraus.

In dem US-Patent 5897955 und der WO 97/46267 A1 wird ein Verfahren beschrieben, wobei die zu modifizierende Oberfläche des Polymers zunächst mit einem Tensid belegt wird, welches dann erst nach Quervernetzung die eigentliche Oberfläche bildet, an die Substanzen gebunden werden können. Auch hier ergibt sich der Nachteil, dass damit keine ausreichende Maskierung der Oberfläche erzielt wird und unspezifische Adsorption nicht unterdrückt werden kann.

Zur Erhöhung der Kompatibilität von Polymeroberflächen wurde vorgeschlagen, an diese Oberflächen asymmetrische Moleküle über radikalische Mechanismen anzubinden. Diese Vorgehensweise ist dadurch an spezifische Materialien gebunden, die zunächst an der Polymeroberfläche adsorbieren und sich anschließend quervemetzen lassen.

Gemäß dem US-Patent 5,263,992 wird die Oberfläche von Biomaterialien zunächst mit einem Verbindungsmolekül in einer radikalischen Reaktion überzogen, wobei das Verbindungsmolekül eine funktionelle Gruppe trägt, an die oberflächenmodifizierende Substanzen gebunden werden. Nachteil des Verfahrens besteht wiederum darin, dass die Adsorption von unerwünschten Substanzen durch diesen Aufbau nicht unterdrückt wird.

US-Patent 5,320,840 beschreibt ein Polymer, welches wasserlöslich ist und damit nicht den Anforderungen an eine feste wasserunlösliche bioabbaubare Matrix genügt. Viele Verfahren, wie zum Beispiel das in US-Patent 5,240,747 beschriebene, erfordern zur Modifizierung von Polymeroberflächen drastische Bedingungen, wie zum Beispiel die Bestrahlung mit UV Licht oder die Präsenz funktioneller Gruppen in Form von Aminogruppen oder Polyaminen (US-Patent 5,399,665 und US-Patent 5,049,403).

In EP 0 844 269 ist ein Blockpolymer mit funktionellen Gruppen an beiden Enden beschrieben, wobei das Blockpolymer aus hydrophoben und hydrophilen Blöcken aufgebaut ist.
Die hydrophilen Blöcke tragen hierbei als funktionelle Gruppen Amino-, Carboxyl- oder Mercaptogruppen, die für eine kovalente Ankopplung von interessierenden oberflächenmodifizierenden Molekülen, die im allgemeinen als funktionelle Gruppen Amino-, Mercapto-, Hydroxylgruppen oder Doppelbindungen aufweisen, zunächst aktiviert werden müssen.

In WO 95/03356 werden nicht lineare Blockcopolymere beschrieben, die aufgebaut sind aus einem multifunktionellen Polymer, an das hydrophile und hydrophobe Polymere angebunden sind. Auch hier erfolgt eine mögliche kovalente Anbindung von modifizierenden Substanzen über eine terminale Hydroxylgruppe des hydrophilen Blocks, z.B. von Polyalkylenglycol, die eine vorhergehende Aktivierung erfordert.

Die oben dargelegten Beispiele zeigen den Bedarf an bioabbaubaren Polymeren, die folgende Eigenschaften besitzen:
1. Ausreichende Maskierung der Polymeroberfläche zur Unterdrückung unspezifischer Adsorption von Substanzen,
2. Unterdrückung der unspezifischen Adhäsion von lebenden Zellen,
3. Vollständige Bioabbaubarkeit und Bioverträglichkeit der Abbauprodukte,
4. Einstellbarkeit der Dichte funktioneller Gruppen auf der Polymeroberfläche, die für die chemische Reaktion mit einer Vielzahl oberflächenmodifizierender Substanzen geeignet sind,
5. Bereitstellung der Möglichkeit, die Polymeroberfläche mit mehreren verschiedenen Substanzen zu belegen,
6. vor oder nach der Verarbeitung zu Formkörpern (zum Beispiel Filme, poröse Schwämme, Mikropartikel, Nanopartikel, Mizellen etc.) eine Bindung der oberflächenmodifizierenden Substanzen zu erlauben, und
7. Bildung von Mustern durch Bindung oberflächenmodifizierender Substanzen auf der Polymeroberfläche.

Um an Polymeroberflächen oberflächenmodifizierende Substanzen dauerhaft zu verankern, sind zwei Voraussetzungen zu erfüllen:
1. Die Polymere müssen an ihrer Oberfläche funktionelle Gruppen tragen, an denen sich die Substanzen chemisch binden lassen.
2. Die funktionellen Gruppen müssen für diese chemischen Reaktionen gut zugänglich sein.

Bekannte bioabbaubare Polymere, wie Poly(α-hydroxy ester) [zum Beispiel Poly(laktid), Poly(laktid-co-glykolid)], Polyanhydride oder Poly(β-hydroxy ester), besitzen zwar geeignete funktionelle Gruppen an beiden Molekülenden, allerdings sind diese Gruppen an der Oberfläche nur schwer zugänglich. Poly(laktid) hat beispielsweise eine Alkohol- und eine Carbonsäurefunktion als Endgruppe, die allerdings aus oben genannten Gründen eine Anbindung an die Polymeroberfläche nicht erlauben.

Zur Lösung der vorstehend genannten Aufgaben wird erfindungsgemäß ein Diblockcopolymer bereitgestellt enthaltend
ein hydrophobes bioabbaubares Polymer a),
ein hydrophiles Polymer b),
mindestens eine reaktive Gruppe c) für die kovalente Anbindung einer oberflächenmodifizierenden Substancz d) an das hydrophile Polymer b), wobei die oberflächenmodifizierende Substanz d) ein Molekül mit einer nukleophilen funktionellen Gruppe ist und von der reaktiven Gruppe c) ohne Aktivierung in wässrigem Medium kovalent gebunden wird, und wobei die mindestens eine reaktive Gruppe c) ausgewählt ist unter 1) einer funktionellen Gruppe und/oder 2) einem mindestens bifunktionellen Molekül mit mindestens einer freien funktionellen Gruppe, wobei die reaktive Gruppe c) die Fähigkeit hat, die oberflächenmodifizierende Substanz d) ohne Aktivierung in wässrigem Medium kovalent zu binden, mit der Maßgabe, dass wenn das hydrophile Polymer b) Polyethylenglykol ist, die reaktive Gruppe c) nicht Hydroxyl ist.

Gemäß einem weiteren Aspekt betrifft die Erfindung ein oberflächenmodifiziertes Diblockcopolymer, das als zusätzliche Komponente eine oberflächenmodifizierende Substanz d) über die reaktive Gruppe c) als Bindeglied gebunden aufweist und Verfahren zu deren Herstellung.

In einer bevorzugten Ausgestaltung liegen die Diblockcopolymere als Formkörper vor.

Weiter betrifft die Erfindung die Anwendung der Diblockcopolymere, nachstehend auch Blockcopolymere genannt, insbesondere im Bereich des drug delivery, drug targeting und vorzugsweise für das tissue engineering.

Gemäß einem weiteren Aspekt betrifft die Erfindung ein Verfahren zur Herstellung eines Blockcopolymers, wobei die Anbindung der mindestens einen Substanz d) an die Oberfläche des Blockcopolymers unter Erzeugung eines Substratmusters erfolgt, und die reaktive Gruppe c) ausgewählt wird unter 1) einem mindesten bifunktionellen Molekül mit mindestens einer freien funktionellen Gruppe und/oder 2) einer funktionellen Gruppe, und damit erhältliche Blockcopolymere.

Aufgrund ihres Aufbaus aus einem hydrophoben und einem hydrophilen Anteil besitzen die erfindungsgemäßen Blockcopolymere einen tensidartigen Charakter. Dieser bewirkt, dass das Polymer zum Beispiel bei Kontakt mit einem wässrigen Medium eine Orientierung erfährt, wobei die hydrophile Komponente b) auf der Polymeroberfläche angereichert vorliegt und dadurch die freie Zugänglichkeit oberflächenmodifizierender Substanzen d) an die reaktive Gruppe c) für die Anbindung ermöglicht.

Gegenstand der Erfindung sind somit Polymere, bei denen ein Teil der Kette, die hydrophile Komponente b) aus der Polymeroberfläche herausragt und für einen ausreichenden Abstand zwischen Polymeroberfläche und reaktiver Gruppe c) sorgt, wodurch die Bindung von oberflächenmodifizierenden Substanzen an die reaktive Gruppe c) erleichtert wird.
Dadurch lassen sich auf einfache Art und Weise spezielle Oberflächen konstruieren und bestmöglich für solche Anwendungen präparieren, bei denen die Oberfläche von Materialien dazu dient, eine bestimmte Funktionalität zu übernehmen.

Gleichzeitig sorgen die erfindungsgemäßen Blockcopolymere für eine Unterdrückung der unspezifischen Adsorption von Molekülen und Adhäsion von Zellen an ihrer Oberfläche.

Eine wichtige Eigenschaft der hier beschriebenen Blockcopolymere ist die vollständige Biokompatibilität der verwendeten Molekülteile, wobei mindestens die hydrophobe Komponente a) biologisch abbaubar ist.
Hierin besteht auch ein Vorteil dieser Polymere im Vergleich zu bereits beschriebenen Systemen zur Modifizierung von Oberflächen, die zum Beispiel auf Polystyrol, Glas oder Metalle zurückgreifen [Mikulec, L.J. and Puleo, D.A. J.Biomed.Mater.Res.32 (1996) 203-208; Puleo, D.A. J.Biomed.Mater.Res.29 (1995) 951-957; Puleo, D.A. Biomaterials17 (1996) 217-222;Puleo, D.A. J.Biomed.Mater.Res.37 (1997) 222-228].
Im Gegensatz zu den genannten Materialien haben die erfindungsgemäßen Blockcopolymere das Potential sich nach Implantation in den menschlichen oder tierischen Körper je nach Bedarf in einer bestimmten Zeit zu zersetzen und den Körper zu verlassen.

Durch die Wahl der Komponenten a) und b) des Blockcopolymers, d.h. die Art und Länge der hydrophoben und der hydrophilen Polymerkette, lassen sich die Materialeigenschaften des Blockcopolymers festlegen. Beispielsweise kann man über die Länge bzw. Struktur der hydrophilen Komponente b) die Beweglichkeit der fixierten Substanz d) variieren. Über die Länge und Struktur der hydrophoben Komponente a) können die Abbaueigenschaften, die mechanische Festigkeit und die Löslichkeit zum Beispiel in Wasser oder einem organischen Lösungsmittel des Copolymers gesteuert werden.

So ist es möglich durch Veränderung der bioabbauren, lipophilen Kette der Komponente a) des Blockcopolymers die Abbauzeit zu verlängern und die mechanische Festigkeit der Polymere zu erhöhen.

Die erfindungsgemäße Ausgestaltung als Blockcopolymer unterstützt die Orientierung, wobei die hydrophile Komponente überwiegend auf der Polymeroberfläche zu liegen kommt und fördert zum Beispiel die Ausbildung von Micellen im wässrigen Medium.

Es zeigen
Abbildung 1 die Anbindung einer oberflächenmodifizierenden Substanz d) an die Oberfläche eines erfindungsgemäßen Blockcopolymers über die reaktive Gruppe c);
Abbildung 2 den Aufbau eines erfindungsgemäßen Blockcopolymers;
Abbildung 3 eine mit unterschiedlichen Substanzen d) belegte Oberfläche eines erfindungsgemäßen Blockcopolymers;
Abbildung 4 Rasterkraftmikroskopische Aufnahmen von erfindungsgemäßen Blockcopolymeren mit unterschiedlichen Gehalt an Polyethylenglykol mit einem Molekulargewicht von 5000 Da und einem Vergleichspolymer ohne PEG;
Abbildung 5 ESCA Spektren der Proteinadsorption auf unterschiedlichen Polymerfilmen;
Abbildung 6 ESCA Spektren der Peptidadsorption auf unterschiedlichen Polymerfilmen;
Abbildung 7 Lichtmikroskopische Aufnahmen von Präadipozyten 3T3-L1 auf unterschiedlichen Polymerfilmen;
Abbildung 8 REM Aufnahmen von mesencymalen Stammzellen aus Ratten auf unterschiedlichen Polymeren;
Abbildung 9 Bestimmung der Aktivität eines erfindungsgemäßen Blockcopolymers über die Anbindung von EDANS und
Abbildung 10 die Anbindung von Trypsin an ein erfindungsgemäßes Polymer.

In den Abbildungen beziehen sich die verwendeten tiefgestellten Indices in den Polymerbezeichnungen auf das Molekulargewicht (Mn) ausgedrückt in kDa.

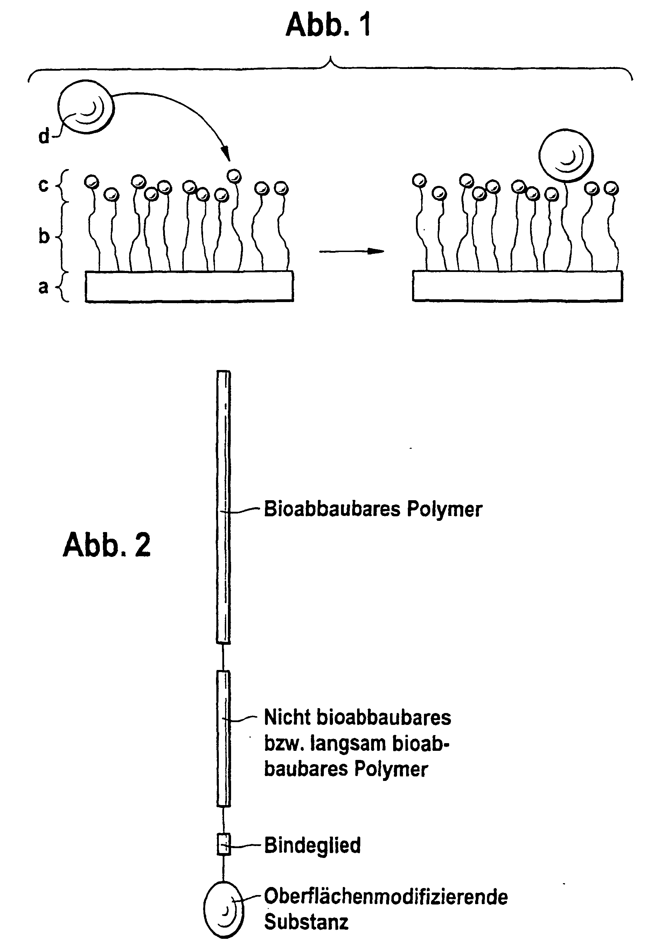

In Abbildung 2 ist ein erfindungsgemäßes oberflächenmodifiziertes Blockcopolymer mit seinen wesentlichen Bauteilen hydrophobe Komponente a), hydrophile Komponente b) und reaktive Gruppe c) sowie oberflächenmodifizierende Substanz d) dargestellt.
Hierbei dient die hydrophobe Komponente a) als Träger und zur Fixierung des gesamten Blockcopolymers, die hydrophile Komponente b) dazu, die reaktive Gruppe c) für die kovalente Anbindung einer oberflächenmodifizierenden Substanz d) verfügbar zu machen und zur Maskierung der Oberfläche und die reaktive Gruppe c) als Bindeglied zur dauerhaften Anbindung der oberflächenmodifizierenden Substanz d).

Das erfindungsgemäße Blockcopolymer kann für die jeweiligen Anwendungen in eine beliebige dafür geeignete Form gebracht werden, wobei die hierbei erhaltenen Formkörper ebenfalls Gegenstand der Erfindung sind.

Das Blockcopolymer kann als zum Beispiel als Film, Partikel in der gewünschten Größe wie zum Beispiel Nano- oder Mikropartikel oder dreidimensionaler Formkörper wie zum Beispiel Monolith vorliegen. Die Formkörper können porös sein. Gemäß einer bevorzugten Ausgestaltung bildet das Blockcopolymer einen porösen Formkörper zum Beispiel in Art eines Schwammes.

Erfindungsgemäß von Vorteil ist, dass das Blockcopolymer bzw. die daraus gebildeten Formkörper für "Instantreaktionen" mit der Substanz d) geeignet sind, was bedeutet, dass sie auf Vorrat im voraus hergestellt und bis zur Anwendung ohne weiteres gelagert werden können, ohne dass sie für die geplante Anwendung erst zeitaufwendig frisch zubereitet werden müssen

Das Blockcopolymer kann aus einem oder mehreren, auch verschiedenen Blöcken aus der hydrophoben a) und hydrophilen Komponente b) aufgebaut sein, wobei die einzelnen Blöcke gleiche Monomere mit gegebenenfalls unterschiedlicher Kettenlänge oder verschiedene Monomere enthalten können.

Die Komponenten a) und b) können gleichzeitig oder unabhängig voneinander linear oder verzweigt, kamm- oder sternförmig sein.
Komponente a) kann auch bei Bedarf eine quervernetzte Verbindung sein.

Die Oberfläche des Blockcopolymers kann mit einer einzigen oder mit verschiedenen Substanzen d) belegt sein, die mindestens eine Substanz d) kann auf der Oberfläche ein beliebiges Muster ausbilden, zum Beispiel kann die Dichte der mindestens einen Substanz d) örtlich konstant oder variabel sein, sie kann einen Gradienten ausbilden usw.
Die Art der Belegung der Oberfläche kann je nach Anwendungsfall gewählt werden. So hat sich gezeigt, dass eine graduelle Belegung mit Wachstumsfaktoren vorteilhaft sein kann.

Als bioabbaubare hydrophobe Komponente a) kann ein beliebiges, für die genannten Applikationen bekanntes bioabbaubares hydrophobes Polymer eingesetzt werden wie sie auch vorstehend bereits genannt worden sind. Weitere Polymere können der Literatur entnommen werden.
Das Polymer für die Komponente a) kann synthetisch, partialsynthetisch oder natürlichen Ursprungs sein.

Sie kann ein Poly(α-hydroxyester (zum Beispiel Polymilchsäure, Polyglykolsäure und deren Copolymeren), Poly(ε-Caprolacton), Poly(β-hydroxyester (wie zum Beispiel Poly(β-Hydroxybutyrat), Poly(β-Hydroxyvalerat)), Poly(dioxanon), Polyäpfelsäure, Polyweinsäure, Polyorthoester, Polycarbonat, Polyamid, Polyanhydrid, Polyphosphazen, Peptid, Polysaccharid, Protein u.a. Polymer sein wie sie in Göpferich A. "Mechanism of Polymer Degradation and Elimination" In: Domb A, Kost JWiseman D, eds. Handbook of Biodegradable Polymers. Harwood acad. publ. Inc., 1997: 451-472; Göpferich A : "Mechanisms of Polymer Degradation and Erosion" Biomaterials 17 *1996a* S.103-114 und Göpfer ich, A. Biomaterials17 (1996a) 103-114; Göpferich, A. Eur.J.Pharm.Biopharm.42 (1996b) 1-11; Leenslag, J.W. et al. Biomaterials (1987) 311-314; Park, K. et al. Biodegradable Hydrogels for Drug Delivery (1993); Suggs, L.J. and Mikos, A.G. (1996) 615-624 beschrieben worden sind.
Weitere geeignete Verbindungen sind zum Beispiel im Handbook of biodegradable Polymers (1997) 451-472 beschrieben.

Vorzugsweise ist das hydrophobe Polymer a) mindestens ein Polymer, das ausgewählt ist unter einem Polyester, Poly-ε-caprolacton, Poly-α-hydroxyester, Poly-β-hydroxyester, Polyanhydrid, Polyamid, Polyphospazen, Polydioxanon, Polyäpfelsäure, Polyweinsäure, Polyorthoester, Polycarbonat, Polysaccharid, Peptid und Protein.

Insbesondere ist die hydrophobe Komponente a) mindestens ein Polymer ausgewählt unter Polylactid, Polyglykolid, Poly(lactid-co-glykolid), Poly-β-hydroxybutyrat und Poly-β-hydroxyvalerat.

Vorzugsweise ist die hydrophobe Komponente a) wasserunlöslich.

Als bioabbaubare Komponente a) sind besonders Polymere geeignet, bei denen der Polymerkettenabbau durch Hydrolyse, enzymatische-, photolytische oder andere Reaktionen herbeigeführt werden kann.

Die minimale Kettenlänge n, gemessen in Monomeren, beträgt n=2, die Obergrenze ergibt sich aus den für das jeweilige Monomer in der Polymerisationsreaktion maximal erreichbaren Molmassen bzw. aus den erwünschten Eigenschaften für das Polymer, d.h. in Abhängigkeit von der beabsichtigten Anwendung.

Im Rahmen der vorliegenden Erfindung beziehen sich die Angaben über die Molmassen (Molekulargewicht), soweit nicht anders angeben, auf das Zahlenmittel Mn.

So kann sich die Kettenlänge der Polymere für die Komponente a) von wenigen bis zu mehreren tausend Monomereinheiten bewegen und das Polymer ein Molekulargewicht von über 10 Millionen Dalton aufweisen kann.
Beispielsweise ist für Polylactid eine obere Grenze der Molmasse von bis zu 100.000 Da bevorzugt.

Wie bereits vorstehend erwähnt entscheidet die Länge der hydrophoben Komponente a) über die Eigenschaften des Blockcopolymers wie der Abbaueigenschaften und der mechanischen Festigkeit.

Beispielsweise führt im Fall einer erfindungsgemäß bevorzugten Kombination von Poly(D,L-lactid) (PLA) als hydrophober Komponente a) und Poly(ethylenglykol) (PEG) für die hydrophile Komponente b) eine Kettenlänge der hydrophoben Komponente a) von ca. n < 20 zu wasserlöslichen Produkten. Ist der PEG Gehalt größer als der PLA Gehalt, dann kann ebenfalls mit wasserlöslichen Produkten gerechnet werden.

Als hydrophile Komponente b) kann ein synthetisches, partialsynthetisches oder natürliches biokompatibles hydrophiles Polymer eingesetzt werden, die auch biologisch abbaubar sein kann.
Sie ist aufgebaut aus mindestens bifunktionalen und vorzugsweise wasserlöslichen Bausteinen.

Beispiele für geeignete Polymere sind Polyethylenglykole, Polyacrylamide, Polyvinylalkohol, Polysaccharide (zum Beispiel modifizierte Cellulosen und Stärken), Alginate, Peptide und Proteine.

Bevorzugte Beispiele für die hydrophile Komponente b) sind Polyethylenglykol, Polypropylenglykol, Polyethylenglykol/Polypropylenglykol-Copolymer, Polyethylenglykol/Polypropylenglykol/Polyethylenglykol-Copolymer, Polybutylenglykol, Polyacrylamid, Polyvinylalkohol, Polysaccharid, Peptid und Protein.

Wird als hydrophile Komponente b) ein symmetrisches Molekül wie zum Beispiel PEG mit zwei gleichen funktionellen Endgruppen, hier Hydroxyl, eingesetzt, sollte bei der Verknüpfung mit der hydrophoben Komponente a) darauf geachtet werden, das die hydrophobe Komponente nicht gleichzeitig mit beiden funktionellen Endgruppen reagiert und damit keine der funktionellen Endgruppen als reaktive Gruppe c) zur kovalenten Anbindung von oberflächenmodifizierenden Substanzen mehr zur Verfügung steht.
Um dieses Problem zu umgehen wird für die Synthese eine hydrophile Komponente b) mit zwei unterschiedlichen funktionellen Endgruppen eingesetzt wie nachstehend am Beispiel des bevorzugt eingesetzten PEGs erläutert wird, wobei diese Ausführungen analog für andere symmetrische Moleküle gelten, die als hydrophile Komponente b) für das erfindungsgemäße Blockcopolymer einsetzbar sind.
So wird im Fall von PEG mit zwei Hydroxylgruppen als Endgruppen eine der Hydroxylgruppen durch eine andere funktionelle Gruppe ersetzt.

Beispielsweise kann Poly(ethylenglykol)-amin (PEG-NH₂) verwendet werden, bei dem eine endständige Hydroxylgruppe durch eine primäre Aminogruppe ersetzt wurde.
Dies gestattet es im Rahmen der Synthese die Anheftung der Monomere der hydrophoben Komponente a) so zu steuern, dass die chemische Reaktion nur an einem Molekülende abläuft.
Die Art der funktionellen Endgruppen ist dabei nicht auf Hydroxylgruppen und Aminogruppen beschränkt. Alternativ lassen sich Thiolgruppen, Doppelbindungen oder Carbonylfunktionen für die Synthese einsetzen. Weitere funktionelle Gruppen sind an sich bekannt und können der Literatur entnommen werden.

Auch die Kettenlänge der hydrophilen Komponente bestimmt sich je nach Anwendung und Bedarf.
Beispielsweise liegt die minimale Kettenlänge für PEG bzw. eines asymmetrischen substituierten PEGs wie zum Beispiel PEG-NH₂ bei einer Ethyleneinheit (Ethanolamin).
Die obere Grenze kann für bestimmte Anwendungen in menschlichen und tierischen Körpern durch das Erfordernis gesetzt sein, dass die freigesetzten Bruchstücke noch nierengängig sein sollten und ausgeschieden werden können.

Geeignete Molmassen insbesondere für die Anwendung im menschlichen oder tierischen Körper liegen vorzugsweise bei 200 bis 10.000 Da, besonders bevorzugt bei 1.000 bis 10.000 Da, wobei insbesondere für Anwendungen außerhalb eines menschlichen oder tierischen Körpers auch Polymere mit höheren Molmassen bis zu mehreren Millionen Da einsetzbar sind.
Vor allem PEG hat sich als besonders geeignet erwiesen eine Polymeroberfläche gegen die Adsorption von Molekülen und die Adhäsion von Zellen zu maskieren.

Erfindungsgemäß besonders bevorzugt sind Blockcopolymere, die aus folgenden Kombinationen zusammengesetzt sind.

Das hydrophobe Polymer a) ist mindestens eines ausgewählt unter Polylactid, Polyglykolid und Poly(lactid-co-glykolid).
Insbesondere bevorzugt ist ein Polylactid, zum Beispiel ein Poly(D,L-Lactid), vorzugsweise mit einer Molmasse in einem Bereich von 1.000 bis 100.000, insbesondere bis 50.000 Da.
Das hydrophile Polymer b) ist ein Polyethylenglykol (PEG), wobei Polyethylenglykole mit einer Molmasse in einem Bereich von 200 bis 10.000 Da, insbesondere 1.000 bis 10.000 Da, besonders bevorzugt sind.

Prinzipiell kann die reaktive Gruppe c) eine beliebige funktionelle Gruppe oder ein mindestens bifunktionelles Molekül sein, die mit der gewählten oberflächenmodifizierenden Substanz d) eine kovalente Bindung ausbilden können, mit der Maßgabe, dass für ein Blockcopolymer nach einem der Ansprüche 1 bis 19 al reaktive Gruppe c) ein mindestens bifunktionelles Molekül eingesetzt wird.

Die erfindungsgemäß eingesetzte reaktive Gruppe c) hat die Fähigkeit, die oberflächenmodifizierende Substanz d) ohne Aktivierung in wässrigem Medium kovalent zu binden.

Die reaktive Gruppe c) kann bestehen aus
einer einzelnen funktionellen Gruppe und damit direkter Aktivierung des hydrophilen Polymers (zum Beispiel aktivierte Säurefunktion oder Epoxid);
physiologischen Dicarbonsäuren (Bernsteinsäure, Weinsäure und Abwandlungen davon wie sie in Anderson, G.W. et al. J.Am.Chem.Soc.86 (1964) 1839-1842] beschrieben sind, die mit Abgangsgruppen (Succinimidylestem) versehen sind, um die Bildung einer bzw. zweier Säureamidgruppierungen zu erreichen;
Dialdehyden (z.B Glutardialdehyd);
Speziellen "Molekülen" für selektive Bindung von Thiolen wie sie in Hermanson, G.T. Bioconjugate Techniques (1996) beschrieben sind, zum Beispiel N-Succinimidyl-3-(2-pyridyldithio)propionat (SPDP) oder Succinimidyl-4-(N-maleimidomethyl)-cyclohexan-1-carboxylat (SMCC);
Photoreaktiven Crosslinkem wie sie in Hermanson, G.T. Bioconjugate Techniques (1996) beschrieben sind, zum Beispiel N-Hydroxysuccinimidyl-4-azidosalicylsäure (NHS-ASA), Sulfosuccinimidyl-2-(p-azidosalicylamido)ethyl-1,3'-dithiopropionat (SASD);
Spaltbaren Crosslinkern wie sie in Hermanson, G.T. Bioconjugate Techniques (1996) beschrieben sind, zum Beispiel Verbindungen aus den oben genannten Gruppen, die sich durch spezielle Reagenzien spalten lassen zum Beispiel Disulfide durch Hydrogenolyse oder durch Disulfidaustausch, Glykolgruppen mit Periodat (zum Beispiel bei der Weinsäure), Estergruppen mit Hydroxylamin; und
Enzymatisch spaltbaren Molekülen wie entsprechende Peptide, zum Beispiel die Sequenz Leu-Gly-Pro-Ala, die von Kollagenase gespalten werden kann, oder Oligosaccharide.

Besonders bevorzugte Beispiele sind reaktive Gruppen c) ausgewählt unter mindestens einer Hydroxylgruppe, Säurechlorid, Ketogruppe, - und insbesondere für den Gegenstand der Ansprüche 1 bis 19 - Dicarbonsäureamid, 3-Maleinimidopropionsäure-N-succinimidylester und Succinimidylester.

Die Synthese der erfindungsgemäßen Blockcopolymere läßt sich prinzipiell auf verschiedenen Wegen bewerkstelligen, wobei gängige Methoden der Polymerchemie verwendet werden.
Zum einen können die Blöcke a) und b) separat synthetisiert und anschließend kovalent gebunden werden. Alternativ dazu ist es möglich eine Polymerkette vorzulegen und die fehlende Kette durch Polymerisation an einem Polymerkettenende zu synthetisieren. So ist es zum Beispiel möglich, Blockcopolymere aus Poly(D,L-lactid) und Poly(ethylenglykol)-amin (PLA-PEG-NH₂) zu synthetisieren, indem man PEG-NH₂ vorlegt und die bioabbaubare PLA Kette durch Ringöffnungspolymerisation aus Dilactid am Hydroxyende des PEG-NH₂ synthetisiert. Prinzipiell ist auch die umgekehrte Vorgehensweise möglich.

Die reaktive Gruppe c) kann hierbei wie im vorstehenden Beispiel im erhaltenen Polymer bereits vorhanden sein bzw. kann bei Bedarf eine in der hydrophilen Komponente b) vorhandene funktionelle Gruppe für die Anbindung der erwünschten oberflächenmodifizierenden Substanz d) zu einer geeigneten reaktiven Gruppe c) umgesetzt oder eingeführt werden.

So kann das Blockcopolymer mit nukleophilen Gruppen modifiziert werden, indem ein mindestens bifunktionelles Molekül wie zum Beispiel Disuccinimidylsuccinat, an eine freie Endgruppe der Komponente b) gekoppelt wird.
Diese Reaktion kann im einfachsten Fall in Lösung stattfinden. Im Falle von PLA-PEG-NH₂ eignet sich zum Beispiel DMSO als Lösungsmittel. Die Reaktion kann auch nach der Verarbeitung des Blockcopolmyers zum Beispiel zu einem geeigneten Formkörper an dessen Oberfläche stattfinden.
Der Vorteil der Aktivierung mit einer reaktiven Gruppe c) besteht darin, dass die Kopplung von vielen oberflächenmodifizierenden Substanzen d) in Wasser abläuft. Durch die reaktive Gruppe c), welche an den hydrophilen Block b) geknüpft wird, endet dieser Block mit einer aktiven Gruppe, die in der Lage ist, andere Moleküle mit nukleophilen funktionellen Gruppen, wie zum Beispiel Aminogruppen, zu binden. Abbildung 1 stellt die Anheftung einer oberflächenmodifizierenden Substanz an eine solche Polymeroberfläche schematisch dar.

Über die im Anschluss stattfindende Anheftung der oberflächenmodifizierenden Substanz d) an den hydrophilen Molekülteil b) kann dann die gewünschte Oberflächeneigenschaft eingestellt werden.

Oberflächenmodifizierende Substanzen d), die für eine Bindung verwendbar sind, sind im allgemeinen solche, die eine nukleophile Gruppe - beispielsweise eine Aminogruppe - tragen, wie zum Beispiel: Kohlenhydrate, unter anderem Mono-, Oligo- und Polysaccharide und Glykoside, Peptide, Proteine, Heteroglykane, Proteoglykane, Glykoproteine, Aminosäuren, Fette, Phospholipide, Glykolipide, Lipoproteine, Arzneistoffe, Antikörper, Enzyme, DNA / RNA, Zellen, die zum Beispiel über auf der Zellmembran befindliche Proteine direkt an das Blockcopolymer anbinden können, aber auch Farbstoffe und molekulare Sensoren.

Beispiele für Peptide sind solche mit dem Motiv -RGD-, IKVAV oder YIGSR und für Proteine Wachstumsfaktoren wie zum Beispiel IGF, EGF, TGF, BMP und Basic FGF, Proteine und Glykoproteine der extrazellulären Matrix wie zum Beispiel Fibronektin, Kollagen, Laminin, bone sialo protein und Hyaluronsäure. Weitere Substanzen sind in der einschlägigen Literatur beschrieben.

Das erfindunggemäße Blockcopolymer eignet sich insbesondere zur Herstellung von drug targeting-Systemen, drug delivery-Systemen, Bioreaktoren, vorzugsweise porösen Formkörpern, für therapeutische und diagnostische Zwecke, für das tissue engineering und als Emulgator.

Nachstehend wird die Anbindung der oberflächenmodifizierenden Substanz allgemein und in bezug auf bevorzugte Anwendungen näher erläutert

Für die Anbindung kann das Blockcopolymer wie die Substanz in Lösung vorliegen oder das Blockcopolymer bildet eine immobilisierte feste Oberfläche, an die die in Lösung vorliegende Substanz d) bindet.
Entscheidender Vorteil der Verwendung des erfindungsgemäßen Blockcopolymers dabei ist, dass die Kopplungsreaktionen unter sehr milden Bedingungen auch im wässrigen Medium durchführbar sind und damit auch empfindliche Substanzen d) angebunden werden können.

So kann man Proteine bei Raumtemperatur und bei einem für das Protein geeigneten pH ohne Denaturierung an der Polymeroberfläche fixieren. Oder man kann Substanzen, die mittels Bestrahlung von Licht an die Oberfläche gebunden werden sollen, in einem beliebigen Lösungsmittel lösen, in welchem das Polymer unlöslich ist. Bei anschließender Bestrahlung mit UV-Licht kann dann die Bindung an die Oberfläche ebenfalls bei Raumtemperatur geknüpft werden.

Für eine Anbindung sind also prinzipiell mehrere Bedingungen denkbar, wobei durch die Verwendung des erfindungsgemäßen Blockcopolymers genügend Freiheiten bestehen, in Bezug auf die Stabilität der Substanz d) und des Polymers optimale Bedingungen zu wählen.
Durch die erfindungsgemäß ermöglichte einfache Art der Anbindung von auch unveränderten, d.h. nicht aktivierten Substanzen d) an das Blockcopolymer mit reaktiver Gruppe c) kann das Verfahren soweit vereinfacht werden, dass es nur nötig ist, den fertig vorgeformten Polymerträger zum Beispiel als Mizellen, Nanopartikel, Polymerfilm oder Polymerschwamm in die Lösung der Substanz d) zu tauchen, um dann nach einer vorgegebenen Reaktionszeit das fertig modifizierte System zu erhalten (Instantreaktion).

Alternativ zur beschriebenen Anbindung der Substanz d) an das Polymer mit reaktiver Gruppe c) ist aber auch der umgekehrte Weg möglich, nämlich zuerst die anzubindende Substanz d) mit der reaktiven Gruppe c) für eine Bindung zu aktivieren, und dann den Komplex aus Substanz d) und reaktiver Gruppe c) über die reaktive Gruppe c) an die Komponente b) des Blockcopolymers aus Komponente a) und b) unter Ausbildung des fertigen oberflächenmodifizierten erfindungsgemäßen Blockcopolymers zu binden.

Hierbei ist jedoch von Nachteil, dass für eine Aktivierung der Substanz d) durch Anbindung der reaktiven Gruppe c) in aller Regel ein größerer Überschuß der reaktiven Gruppe c) erforderlich ist, um die Bildung von Dimeren zu verhindern. Dieser muß jedoch nach der Aktivierung wieder entfernt werden. Das hat zur Folge, dass sich, vor allem bei ebenfalls niedermolekularen Substanzen d), die Aufreinigung schwieriger gestaltet.

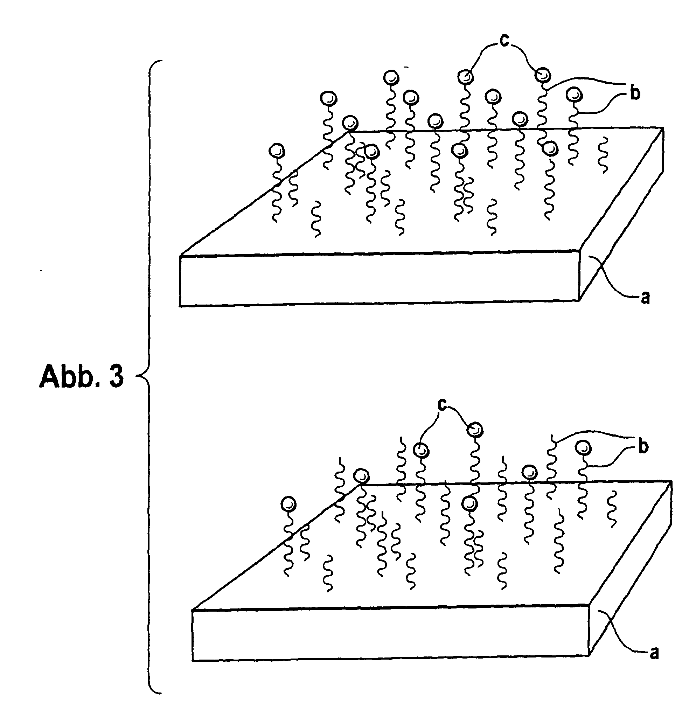

Zusätzlich zur Herstellung von homogen belegten Oberflächen sind mit den erfindungsgemäßen Blockcopolymeren auch nicht homogen belegte Flächen einfach herzustellen. Das bedeutet, dass man beispielsweise Gradienten oder Muster (pattern) der oberflächenmodifizierenden Substanzen d) auf diesen Polymeren erzeugen kann. Das kann durch punktuelles Aufbringen der Substanzen d) (zum Beispiel mit einem Tintenstrahlverfahren) oder aber durch punktuelle Aktivierung der reaktiven Gruppen c) durch Bestrahlung (zum Beispiel mit UV-Licht), Beschuss mit Teilchen, Stempeln oder Soft Lithographie geschehen.
So können strukturierte Oberflächen geschaffen werden, die es auch erlauben, beliebige Kombinationen von Substanzen d) zum Beispiel auf ihre Wirkung auf Zellen zu untersuchen bzw. um Kombinationen von Zellen in ganz spezieller räumlicher Orientierung zueinander zu züchten oder aber auch um mit Enzymen biotechnologische Miniaturfabriken zu konstruieren, die in einem gekoppelten Verfahren spezielle Reaktionen durchführen. In Abbildung 3 sind solche Oberflächen dargestellt, die sich durch zwei unterschiedliche Substanzen d) und zudem noch einer inerten kürzeren Komponente auszeichnen.

Im Rahmen des tissue engineering ist es möglich die Adhäsion, Proliferation und Differenzierung von Zellen besser als bisher zu beeinflussen, da durch die erfindungsgemäßen Blockcopolymere eine exakte Belegung der Oberfläche mit einem oder mehreren Substanzen d) möglich ist. Gleichzeitig wird die unspezifische Wechselwirkung von unerwünschten Substanzen d) insbesondere unerwünschten Zellen, mit den Polymeroberflächen unterdrückt.

Im Rahmen des drug delivery ist es möglich, die Polymere zur Oberflächenmodifizierung einzusetzen, welche kleine Polymerpartikel an spezifische Gewebe oder Organe verteilt (drug targeting). Dies wird durch Bindung von spezifischen Substanzen d) wie zum Beispiel Plasmaproteinen, Antikörpern oder Lektinen erreicht. Weitere hierfür mögliche Substanzen d) sind in der einschlägigen Literatur beschrieben sind.

Eine weitere Anwendung besteht in der chemischen Bindung der Polymere in Form von Partikeln an Gewebe (bioadhäsive Systeme). Durch diese Anwendung kann ein Wirkstoff in erhöhter Konzentration an das Zielgewebe verteilt werden.

Durch den Polymerabbau ist zu erwarten, dass die am hydrophilen Polymerblock b) angeheftete Substanz d) im Rahmen der Hydrolyse freigesetzt wird. Dieser dynamische Vorgang gestattet die zeitlich gesteuerte Veränderung der Oberflächeneigenschaften des erfindungsgemäßen Blockcopolymers.

Die erfindungsgemäßen Polymere lassen sich auch zu diagnostischen Zwecken einsetzen, indem auf ihrer Oberfläche Substanzen d) gebunden werden, die mit den zu analysierenden Molekülen eine Bindung eingehen. Das Analysat kann dann zusammen mit dem Polymer (zum Beispiel über einen geeigneten Formkörper) von der Probe abgetrennt werden.

Zur näheren Veranschaulichung der Erfindung ist im folgenden die Herstellung eines erfindungsgemäßen Blockcopolymers sowie die anschließende Anbindung eines Proteins am Beispiel des PEG-PLAs dargestellt.

### 1. Beispiel: Herstellung von aktivem SWS-NH-PEG-PLA

### a) Synthese von NH₂-PEG

Die Herstellung erfolgte nach Yokoyama, M. et al. Bioconjug.Chem.3 (1992) 275-276.
Die gewünschte Menge Ethylenoxid wurde bei -79°C (Trockeneis + Methanolbad) in getrocknetes THF in einem Dreihalskolben eingeleitet und darin gelöst. Die Ethylenoxidflasche wurde nach dem Einleiten zurückgewogen und damit die vorgelegte Menge Ethylenoxid bestimmt. Entsprechend des gewünschten Molekulargewichts des Polymers wurde nun die berechnete Menge 0,5M Lösung von Kalium-bis-(trimethylsilyl)-amid in Toluol aus einem Tropftrichter zugegeben.

Das Reaktionsgemisch wurde dann bei 20°C in dem verschlossenen Dreihalskolben 36 Stunden gerührt. Die so erhaltene Polymerlösung wurde in die 12fache Menge an Ether getropft und das gefällte Polymer daraus abfiltriert. Nach Auflösen des erhaltenen Polymers in THF wurde eine kleine Menge 0,1N Salzsäure zugegeben und damit das Silylamid gespalten. Die so erhaltene Lösung des fertigen Endprodukts wurde 5 Min bei Raumtemperatur gerührt und erneut in Ether gegeben, um das reine Polymer zu präzipitieren.

### b) Synthese von NH₂-PEG-PLA

Die Synthese erfolgte nach Kricheldorf, H.R. and Kreiser-Saunders, I. Macromol.Symp.103 (1996) 85-102;Leenslag, J.W. and Pennings, A.J. Makromol.Chem.188 (1987) 1809-1814.
Die Ausgangsprodukte der Synthese, das gemäß 1a) synthetisierte NH₂-PEG und cyclisches DL-Dilactid (3,6-Dimethyl-1,4-dioxan-2,5-dion), wurden in den gewünschten Gewichtsverhältnissen in je einen Rundkolben gegeben und in Toluol p.A. aufgelöst. Dazu wurden beide Kolben am Wasserabscheider erhitzt, um das noch im Toluol vorhandene Wasser zu entfernen. Die so erhaltenen Lösungen wurden dann im Dreihalskolben vereinigt und unter permanentem Stickstoffstrom erneut erhitzt.

Zu dem siedendem Reaktionsgemisch wurde dann der abgewogene Katalysator (Zinn-2-ethylhexanoat) gegeben und dann das Gemisch 8 Stunden am Sieden gehalten.
Die so erhaltene Polymerlösung wurde nach dem Abkühlen in einen Rundkolben überführt und dreimal mit Dichlormethan am Rotationsverdampfer bis zur Trockene abrotiert. Nach zweimaligem Abrotieren nach Zugabe von Aceton wurde das so erhaltene Polymer erneut in Aceton gelöst und in eisgekühltes demineralisiertes Wasser eingetopft und dadurch gefällt. Die so erhaltenen Polymerfäden wurden durch ein Filter abgetrennt und zum Trocknen in einen Vakuumtrockenschrank gegeben. Die Bestimmung der Molekülmasse kann durch GPC erfolgen.

### c) Synthese des Disuccinimidylesters der Weinsäure (DSWS)

Die Synthese erfolgte nach Anderson, G.W. et al. J.Am.Chem.Soc.86 (1964) 1839-1842.
Die berechneten Mengen Weinsäure und N-Hydroxysuccinimid wurden in einem Rundkolben in einer Mischung aus Dioxan und Ethylacetat (4:1) gelöst. Zu dieser Lösung wurde die Lösung des Katalysators (Dicyclohexylcarbodiimid) im gleichen Lösungsmittelgemisch gegeben und das Ganze bei 0°C 20h im Eisbad gerührt. Der so erhaltene Nierderschlag wurde abfiltriert und mit Dioxan gewaschen. Aus diesem Niederschlag wurde das Endprodukt durch vorsichtiges Erwärmen mit Acetonitril extrahiert. Die so erhaltene Lösung wurde am Rotationsverdampfer eingeengt und das Produkt im Vakuumschrank getrocknet.

### d) Synthese von SWS-NH-PEG-PLA

Die gemäß 1c) und 1b) erhaltenen Ausgangsprodukte Disuccinimidylweinsäure und NH₂-PEG-PLA wurden mit einem leichten Überschuß des Diesters in Acetonitril gelöst und mit einigen Tropfen Triethylamin versehen. Nach kurzem Erhitzen zum Sieden wurde die Mischung für 24h gerührt. Das Endprodukt wurde durch Abrotieren vom Acetonitril getrennt und in Aceton aufgelöst. Die so erhaltene Polymerlösung wurde in Wasser eingetropft und der Niederschlag abfiltriert. Nach Trocknen im Vakuum steht das fertige aktive Polymer zur Verfügung.

Nach der vorstehend beschriebenen Vorgehensweise wurden für die nachstehenden Untersuchungen NH₂-PEG-PLA-Diblockcopolymere mit unterschiedlichen Molekülmassen für die Komponenten a) und b) hergestellt bzw. in analoger Weise mit Methylgruppen inaktivierte Polymere, bei denen die reaktive Gruppe c) durch eine Methylgruppe ersetzt war.

### Beispiel 2

### Herstellung von Amino-Polyethylenglycol-Poly-L-lactid (NH₂-PEG-PLLA)

Es wurde im wesentlichen wie in Beispiel 1b) verfahren.
Jedoch wurde anstelle des cyclischen D,L-Dilactids das cyclische L-Dilactid eingesetzt.
Weiter wurde das erhaltene Polymer nach dem dreimaligen Abrotieren mit Dichlormethan erneut in Dichlormethan gelöst und in eisgekühlten Diethylether getropft. Die so erhaltenen Polymerfäden wurden durch ein Filter abgetrennt und zum Trocknen in einen Vakuumtrockenschrank gegeben.
Die Bestimmung des Molekulargewichts erfolgte durch GPC und die des anzahlmittleren Molekulargewichts auch durch ¹H-NMR über die Berechnung der Integrale erfolgen.

### Beispiel 3

### Anknüpfung von oberflächenmodifizierenden Substanzen d)

Die Anbindung von oberflächenmodifiezierenden Substanzen kann nach in Hill, M. et al. FEBS Lett.102 (1979) 282-286;Schulman, L.H. et al. Nucleic.Acids.Res.9 (1981) 1203-1217 beschriebenen Verfahren erfolgen.

Die Anknüpfung der oberflächenmodifizierenden Substanzen d) an das nach Beispiel 1 erhaltene erfindungsgemäße Blockcopolymer kann prinzipiell auf zwei Weisen geschehen. Zum einen ist es möglich, die Substanz d) und das Blockcopolymer in Lösung zu verbinden, wenn die Substanz d) die nachfolgenden Verarbeitungsschritte unbeschadet übersteht. Oder zum anderen kann erst das Blockcopolymer zu der gewünschten Form verarbeitet und dann erst die Substanz d) angeknüpft werden. In beiden Fällen sollte durch Pufferung sichergestellt werden, dass zum Beispiel eine Aminogruppe unprotoniert vorliegt, um möglichst quantitative Ausbeuten zu erhalten. Mit einer Pufferung kann zudem noch der Ort der Anbindung an die Substanz d) gesteuert werden, wenn man den pH so wählt, daß zum Beispiel nur eine Aminogruppe unprotoniert vorliegt.

### Beispiel 4

### Charakterisierung von Polymerfilmen - Eigenschaften der Blockcopolymere

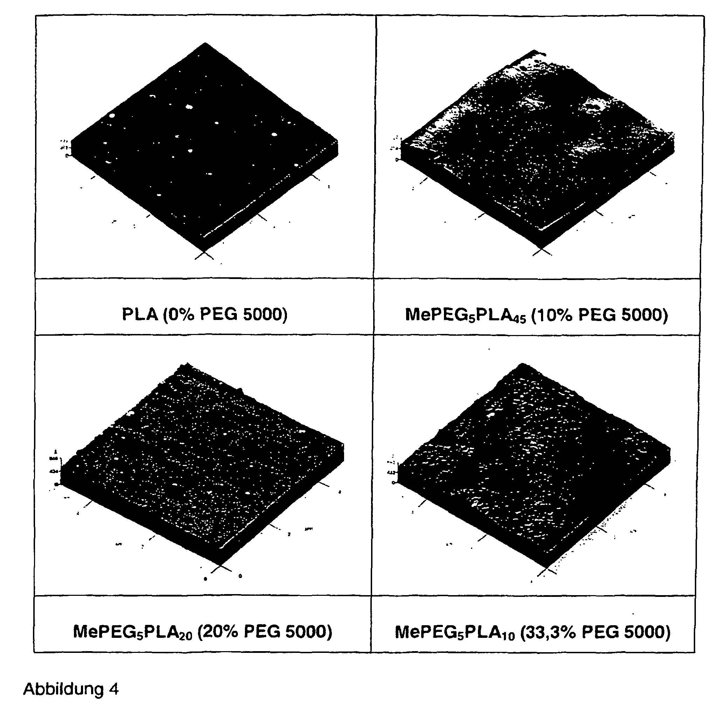

4a) Untersuchung der Blockcopolymere mit AFM
   Zur Charakterisierung der Oberflächentopographie der Blockcopolymere wurde die Rasterkraftmikroskopie verwendet. Dazu wurden die Polymere in einer 5%igen Lösung in Chloroform mittels Spincasting auf quadratische Metallplättchen (5x5mm) aufgebracht und anschließend getrocknet. Die so erhaltenen Filme wurden dann mit AFM untersucht.
   Die Ergebnisse sind in Abbildung 4 gezeigt:
   Was man erhält, sind je nach untersuchtem Polymer unterschiedliche Dichten buckliger Erhebungen auf der Polymeroberfläche. Bei den Erhebungen handelt es sich um Kristallite des Polyethylenglykols, die mit ansteigendem Gehalt an Polyethylenglykol im Blockcopolymer zunehmen. Das heißt, die Polymere zeichnen sich aus durch eine Phasentrennung der Blöcke und damit einer Verfügbarkeit der hydrophilen Ketten an der Polymeroberfläche.
4b) Untersuchung der Proteinadsorption
   Die Untersuchung der Proteinadsorption und deren Unterdrückung erfolgte an verschiedenen erfindungsgemäß einsetzbaren PEG-PLA-Blockcopolymeren, die anstelle einer reaktiven Gruppe c) eine Methylgruppe enthielten und somit für die Proteinanbindung inaktiviert waren.

Zur Untersuchung der Adsorption von Proteinen an die Polymerfilme wurden derartige inaktive Polymere auf Metallplättchen (0,5x0,5 cm) ausgegossen und intensiv getrocknet (mindestens 2 Tage im Vakuum), die so erhalten Filme wurden dann mit den zu untersuchenden Proteinlösungen inkubiert und nach mehrmaligem Abwaschen mit phosphatgepufferter (pH=7,4) isotoner Kochsalzlösung abgewaschen. Die so erhaltenen Filme wurden dann erneut getrocknet und mit ESCA vermessen.

Als Modelsubstanzen wurden foetales Rinderserum, atriales natriuretisches Peptid und Lachs-Calcitonin eingesetzt.

Die ESCA Spektren dienten zur Quantifizierung des adsorbierten Proteins bzw. Peptids, da durch die Aminosäuren des adsorbierten Proteins nun auch Stickstoff auf der Polymeroberfläche zu finden ist. Als Vergleich wurden Polymerfilme aus reiner Polymilchsäure sowie nicht inkubierte Polymerfilme verwendet.

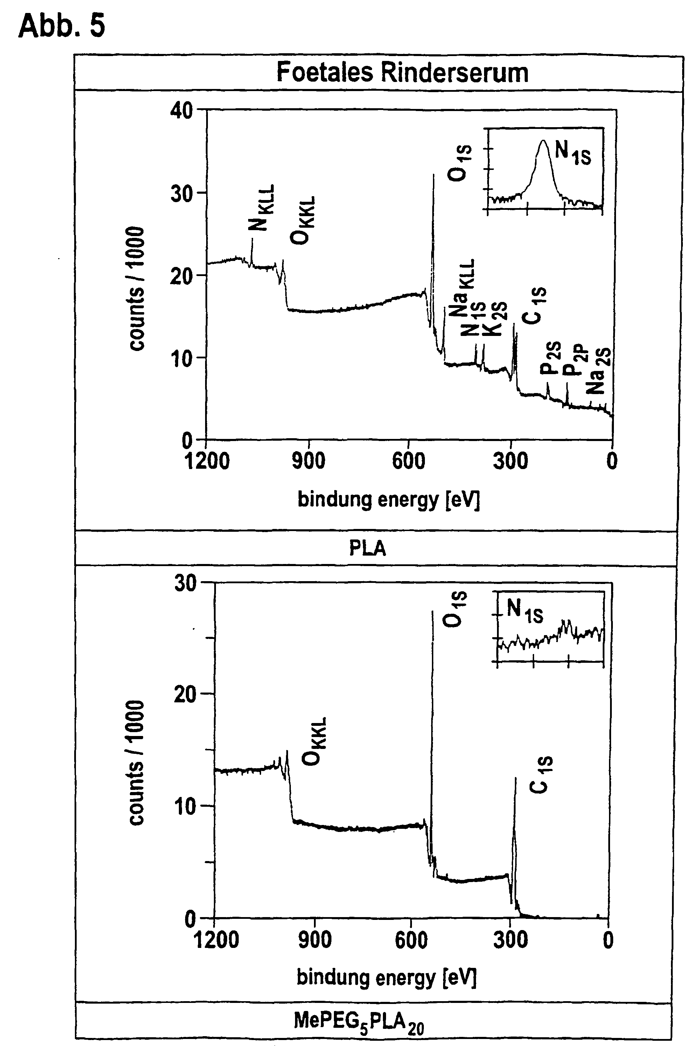

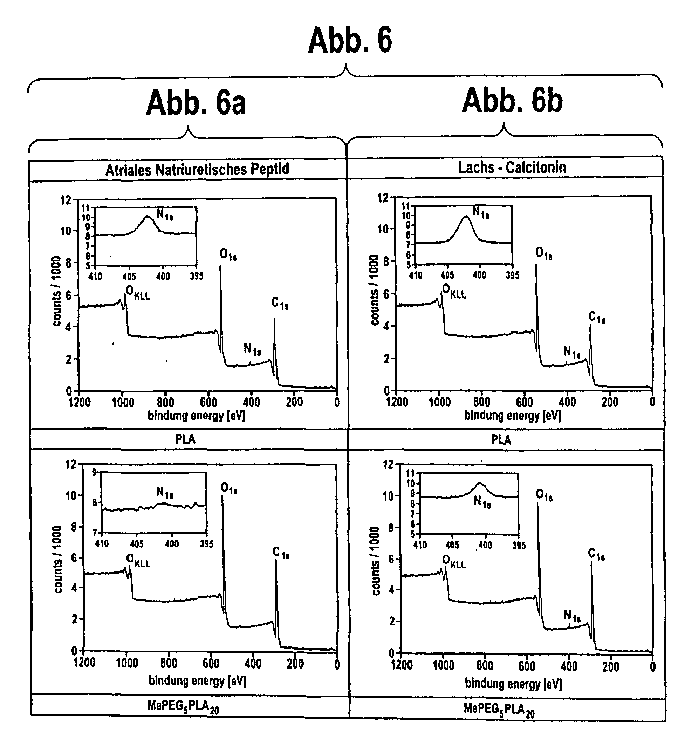

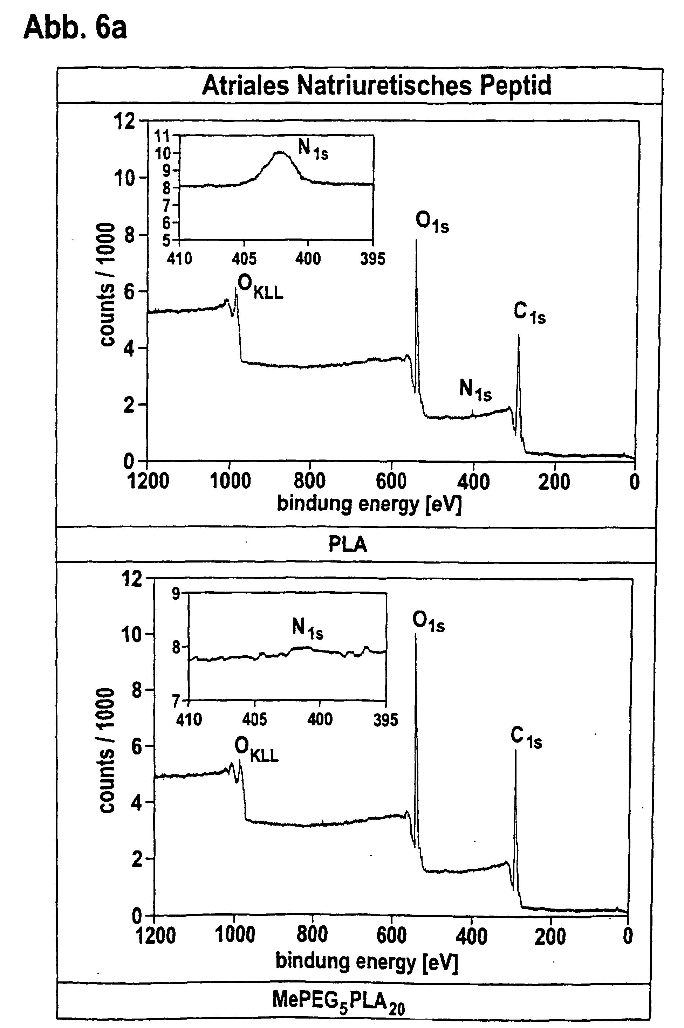

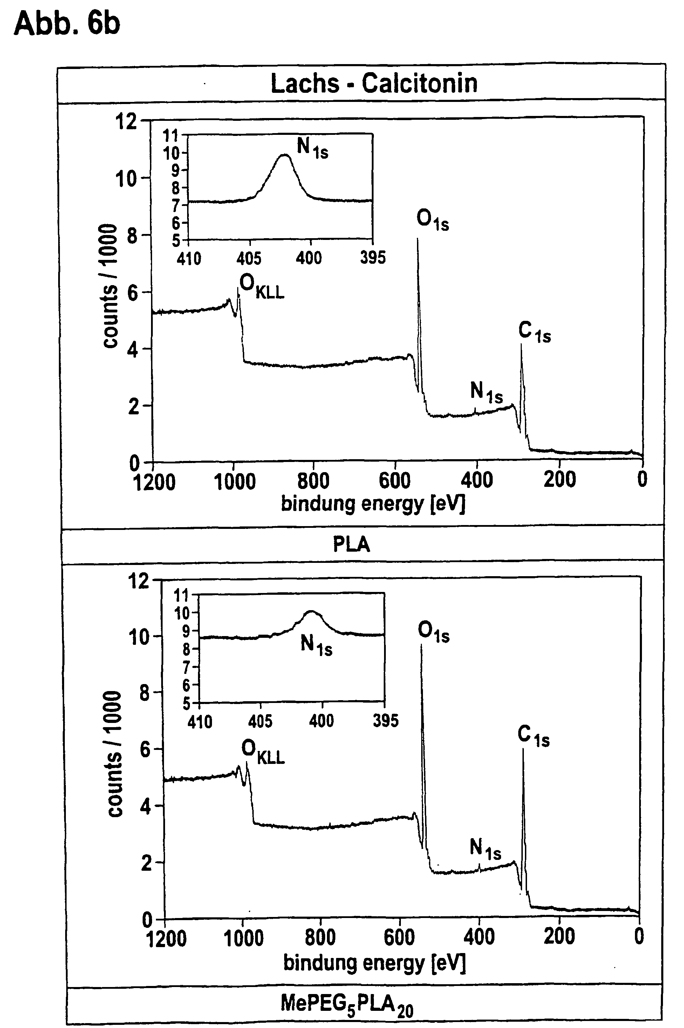

Die Ergebnisse sind in Abbildungen 5 und 6 gezeigt.
Es wird eine von der Art der jeweiligen verwendeten oberflächenmodifizierenden Substanz d) abhängige Unterdrückung der Adsorption beobachtet.
So wird die Adsorption von fötalem Rinderserum durch Einbringen einer hydrophilen Kette im Rahmen der Messgenauigkeit vollständig unterdrückt (siehe Abbildung 5). Bei den Modellpeptiden Calcitonin und atrialen natriuretischen Peptid (ANP) ist teilweise noch eine geringe Adsorption von Peptid zu identifizieren (siehe Abbildung 6).
Im Ergebniss kann somit festgestellt werden, dass die erfindungsgemäß einsetzbaren Blockcopolymere die Adsorption von Proteinen und Peptiden zu steuern vermögen und damit Einfluss nehmen können auf das Verhalten von Zellen, die mit der modifizierten Polymeroberfläche in Kontakt kommen.

### Beispiel 5

### Untersuchung des Adhäsionsverhaltens gegenüber Zellen

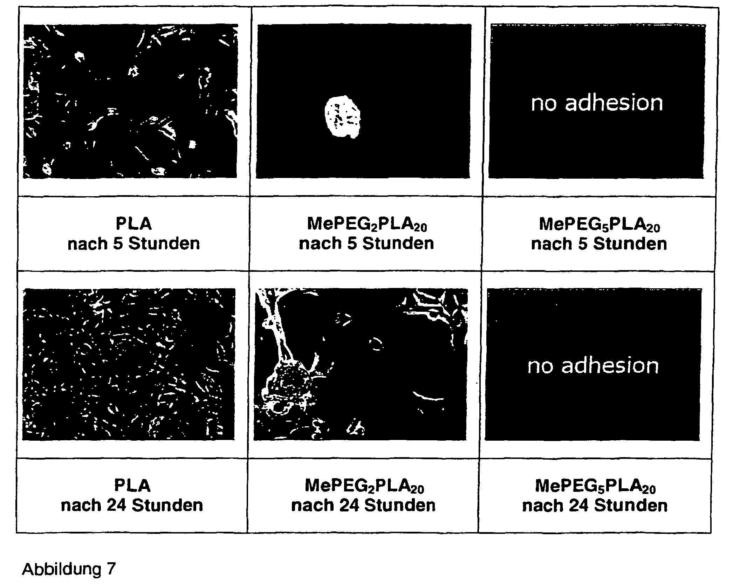

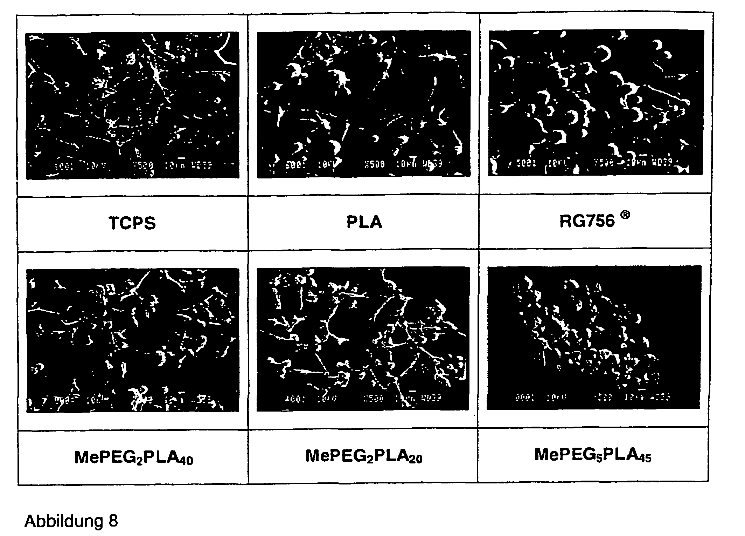

5a) Zellen aus einer Präadipozytenzelllinie wurden in einer Suspension auf ausgegossene Filme aus unterschiedlichen Polymeren gegeben und ihre Adhäsion nach 5 und nach 24 Stunden beurteilt. Dazu wurden die Suspensionen vor dem Mikroskopieren mit Puffer abgewaschen, und somit nur die fest adhärierten Zellen beobachtet.
   Die Ergebnisse sind in Abbildung 7 gezeigt.
   Was man erkennen kann, sind Unterschiede im Zellverhalten je nachdem welche Polymere verwendet wurden. So kann man beispielsweise auf dem MePEG₅PLA₂₀ sowohl nach 5 als auch nach 24 Stunden keine adhärierten Zellen erkennen, wobei auf dem Blockcopolymer MePEG₂PLA₂₀ mit der kürzeren PEG-Kette im geringen Ausmaß Zellen zu erkennen sind, die jedoch im Vergleich zur Probe aus lipophiler Polymilchsäure nur schlecht adhärieren. Man findet hier nach 5 Stunden nur locker gebundene Zellaggregate und erst nach 24 Stunden vereinzelt schon gespreitete, d.h. fester gebundene Zellen.
   Im Ergebnis ist jedoch festzuhalten, dass die erfindungsgemäßen Blockcopolymere die Adhäsion von Zellen unterdrücken bzw. reduzieren können und somit unspezifische Wechselwirkungen verhindern oder gering halten können.
5b) Zur Untersuchung der Adhäsion von Stammzellen von Ratten wurden dünne Polymerfilme aus unterschiedlichen mit Methyl inaktivierten erfindungsgemäßen Blockcopolymeren (Me-PEG₂-PLA₂₀, ME-PEG₂-PLA₄₀ und Me-PEG₅-PLA₄₅) und zum Vergleich aus PLA, TCPS (Tissue Culture Polystyrol) sowie RG756 (einer Marke für Poly(D,L-Lactid-co-glykolid 75:25) auf Polypropylenscheiben ausgegossen. Auf diese Filme wurden dann die Knochenmarksstammzellen von 6 Wochen alten männlichen Sprague Dawley Ratten mit einer Dichte von 5000 Zellen pro cm² ausgesät. Nach 3 Stunden wurde dann die Morphologie der adhärierten Zellen mit dem Rasterelektronenmikroskop betrachtet.
   Die erhaltenen Ergebnisse sind in Abbildung 8 dargestellt.

Die Anzahl der Zellen wurde zusätzlich durch Auszählen mit dem Lichtmikroskop ermittelt.
Es zeigte sich, dass auf den erfindungsgemäß einsetzbaren Blockcopolymeren die Zellzahl umso geringer war, je größer die hydrophile Komponente b) des Polymers war. Des weiteren zeigten die Rasterelektronenmikroskopischen Bilder, dass gegebenenfalls auf den erfindungsgemäßen Blockcopolymeren adhärierte Zellen abgerundeter als auf den Vergleichspolymeren aus nur hydrophoben Bestandteilen waren, was ein deutliches Zeichen für die geringe Adhäsionsneigung der Zellen an die Polymeroberfläche ist.

### Beispiel 6

### Charakterisierung der aktiven Polymere hinsichtlich ihrer Bindungsfähigkeiten

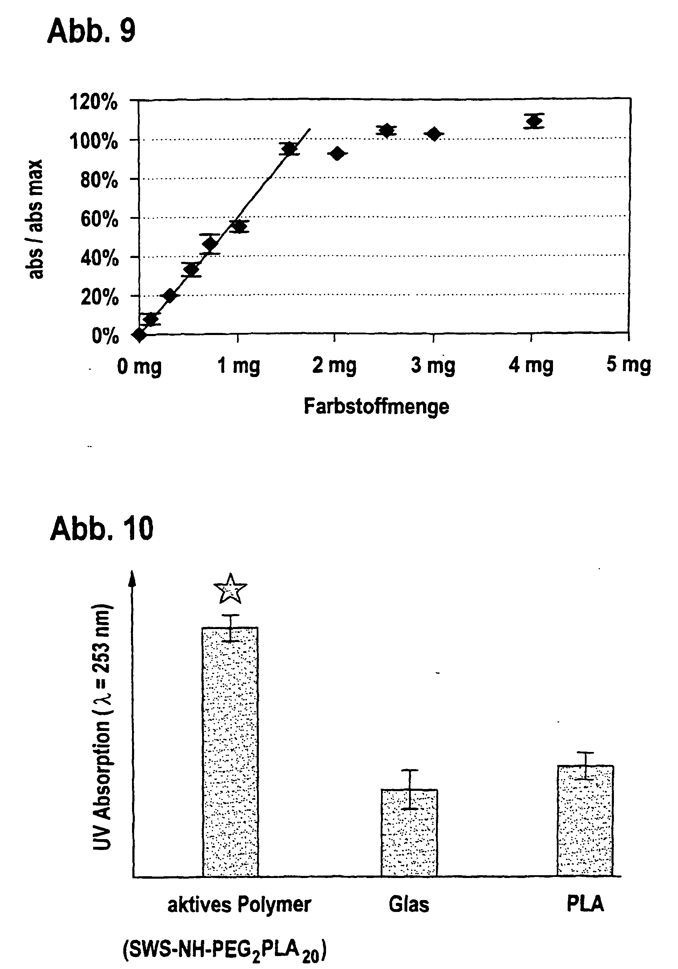

6a) Nachweis der Bindungsfähigkeit mit einfachen Modelsubstanzen mit Aminogruppe in Lösung
   Zur Untersuchung der Reaktivität in Lösung wurde eine bestimmte Menge Polymer (SWS-NH-PEG₂-PLA₂₀) (50 mg) in 2000 µl Dimethylformamid (DMF) gelöst und mit einer bestimmten Menge Farbstoff (EDANS, 5-((2-aminoethyl)amino)naphthalin-1-sulfonsäure, Natrium Salz, 0,1 - 4 mg), der ebenfalls in DMF gelöst war, versetzt. Um mögliche Protonierungen der Aminogruppe auszuschließen wurden 20 µl Triethylamin als Protonenfänger zugegeben. Die so erhaltene Lösung wurde dann über Nacht im Schüttler bei 37°C inkubiert. Nach der Reaktionszeit wurden dann 200 µl der Lösung mit 1800 µl Chloroform verdünnt und der überschüssige ausfallende Farbstoff durch Filtration abgetrennt. 200 µl der klaren Lösungen wurden dann mittels Gelpermeationschromatographie vermessen. Die Menge an kovalent gebundenem Farbstoff wurde über die Zunahme der UV-Absorption bei 335 nm bestimmt.
   Das Ergebnis ist in Abbildung 9 gezeigt.
   Wertet man die erhaltenen Flächen aus, so erhält man ein Diagramm, bei dem man mit steigender Farbstoffmenge einen Anstieg der Peakfläche beobachten kann. Ab einer bestimmten Farbstoffmenge erhält man dann ein Plateau, was durch die beschränkte Anzahl an reaktiven Gruppen bedingt ist. Über diese Bestimmung lässt sich einfach die Menge reaktiver Gruppen in einer Polymercharge bestimmen.
6b) Nachweis der Bindungsfähigkeit mit einfachen Modelsubstanzen mit Aminogruppe an festen Polymeroberflächen
   Die Aktivität an festen Oberflächen kann ebenso wie die Aktivität in Lösung untersucht werden.
   Dazu wurden Filme eines erfindungsgemäßen aktiven Blockcopolymers (SWS-NH-PEG₂-PLA₂₀), die auf runde Glasdeckgläser ausgegossen waren, mit einer wässrigen Lösung des Farbstoffs (5-Aminoeosin) überschichtet und diese Lösung dann zwei Stunden einwirken gelassen. Die so erhaltenen markierten Filme wurden mehrmals mit Phosphatpuffer gewaschen und anschließend getrocknet. Die getrockneten Filme wurden dann in Chlorform gelöst und dann mittels GPC gegebenenfalls adsorbierter von kovalent gebundenem Farbstoff abgetrennt.
   Beobachtet wurde das Vorhandensein einer erhöhten UV-Absorption bei dem Molekulargewicht der Polymere. Diese UV-Absorption ist zu erklären durch eine kovalente Verbindung zwischen Farbstoff und Polymer.
6c) Anbindung von Proteinen
   Zur Untersuchung der Fähigkeit auch komplexere Verbindungen wie Proteine zu binden, wurde das Enzym Trypsin als Modellsubstanz verwendet.
   Zur Anbindung des Enzyms an Polymerfilme wurden auf runden Glasdeckgläsem ausgegossene Filme der unterschiedlichen Polymere (SWS-NH-PEG₂-PLA₂₀ und zum Vergleich PLA) mit Lösungen des Enzyms Trypsin in phosphatgepufferter isotoner Kochsalzlösung (PBS-Puffer) inkubiert. Die hierzu verwendeten Konzentrationen des Enzyms betrugen 0,5 bzw. 1,0 mg/ml.
   Die so erhaltenen Trypsin gekoppelten Polymere wurden dann nach 2 Stunden Inkubationszeit mit 0,05% Tween 20 enthaltenen PBS-Puffer 3 mal gewaschen, um gegebenenfalls adsorbiertes Protein so gut wie möglich zu entfernen. Die so gewaschenen Filme wurden dann trocken getupft und in Sixwellplates überführt.
   In die einzelnen Wells der Platten wurde dann jeweils 2 ml des Reaktionsmediums zugegeben und die enzymatische Reaktion bei 37°C für 2 Stunden im Inkubator durchgeführt. Bei dem Reaktionsmedium handelte es sich um eine 1 millimolare Lösung von Benzoyl-L-Argininethylester (BAEE) in Tris - Puffer mit pH = 8,0. Nach 2 Stunden wurde die enzymatische Reaktion durch Zugabe einer wässrigen Lösung eines Trypsininhibitors aus Sojabohnen gestoppt und damit die Umwandlung des Enzymsubstrats beendet. Die so erhaltenen Lösungen wurden dann bei 253 nm UV-photometrisch vermessen.

Das Ergebnis ist in Abbildung 10 gezeigt:
Der Vergleich mit PLA und mit den reinen Glasdeckgläsem zeigt einen deutlichen Anstieg der Substratumsetzung im Falle des erfindungsgemäßen Blockcopolymers, die durch die Menge an kovalent gebundenem Enzym bedingt ist.

## Patentansprüche

1. Diblockcopolymer enthaltend
ein hydrophobes bioabbaubares Polymer a),
ein hydrophiles Polymer b),
mindestens eine reaktive Gruppe c) für die kovalente Anbindung einer oberflächenmodifizierenden Substanz d) an das hydrophile Polymer b), wobei die oberflächenmodifizierende Substanz d) ein Molekül mit einer nukleophilen funktionellen Gruppe ist und von der reaktiven Gruppe c) ohne Aktivierung in wässrigem Medium kovalent gebunden wird, und wobei die mindestens eine reaktive Gruppe c) ausgewählt ist unter 1) einer funktionellen Gruppe und/oder 2) einem mindestens bifunktionellen Molekül mit mindestens einer freien funktionellen Gruppe, wobei die reaktive Gruppe c) die Fähigkeit hat, die oberflächenmodifizierenden Substanz d) ohne Aktivierung in wässrigem Medium kovalent zu binden, mit der Maßgabe, dass wenn das hydrophile Polymer b) Polyethylenglykol ist, die reaktive Gruppe c) nicht Hydroxyl ist.

2. Diblockcopolymer nach Anspruch 1,
**dadurch gekennzeichnet,**
**dass** das hydrophobe Polymer a) und/oder hydrophile Polymer b) ausgewählt sind unter einem linearen und/oder verzweigten Polymer.

3. Diblockcopolymer nach einem der vorhergehenden Ansprüche,
**dadurch gekennzeichnet,**
**dass** das hydrophobe Polymer a) mindestens ein Polymer ausgewählt unter Polyester, Poly-ε-caprolacton, Poly-α-hydroxyester, Poly-β-hydroxyester, Polyamid, Polyphosphazen, Polyanhydrid, Polydioxanon, Polyäpfelsäure, Polyweinsäure, Polyorthoester, Polycarbonat, Peptid, Polysaccharid und Protein ist.

4. Diblockcopolymer nach Anspruch 3,
**dadurch gekennzeichnet,**
**dass** das hydrophobe Polymer a) mindestens ein Polymer ausgewählt unter Polylactid, Polyglykolid, Poly(lactid-co-glykolid), Poly-β-hydroxybutyrat und Poly-β-hydroxyvalerat ist.

5. Diblockcopolymer nach einem der vorhergehenden Ansprüche,
**dadurch gekennzeichnet,**
**dass** das hydrophile Polymer b) mindestens ein Polymer ausgewählt unter Polyethylenglykol, Polypropylenglykol, Polyethylenglykol/Polypropylenglykol-Copolymer, Polyethylenglykol/Polypropylenglykol/ Polyethylenglykol-Copolymer, Polybutylenglykol, Polyacrylamid, Polyvinylalkohol, Polysaccharid, Peptid und Protein ist.

6. Diblockcopolymer nach einem der vorhergehenden Ansprüche,
**dadurch gekennzeichnet,**
**dass** die reaktive Gruppe c) mindestens eine ausgewählt unter einer Ketogruppe, einem Säurechlorid, Dicarbonsäureamid, 3-Maleinimidopropionsäure-N-succinimidylester und Succinimidylester ist.

7. Diblockcopolymer nach einem der vorhergehenden Ansprüche,
**dadurch gekennzeichnet,**
**dass** das hydrophobe Polymer a) mindestens eines ausgewählt unter Polylactid, Polyglykolid und Poly(lactid-co-glykolid) ist.

8. Diblockcopolymer nach Anspruch 7,
**dadurch gekennzeichnet,**
**dass** das hydrophile Polymer b) Polyethylenglykol ist.

9. Diblockcopolymer nach Anspruch 8,
**dadurch gekennzeichnet,**
**dass** das Polyethylenglykol eine Molmasse in einem Bereich von 200 bis 10,000 Da aufweist.

10. Diblockcopolymer nach einem der vorhergehenden Ansprüche,
**dadurch gekennzeichnet,**
**dass** das hydrophobe Polymer a) Polyactid vorzugsweise mit einer Molmasse in einem Bereich von 1,000 bis 100,000 Da ist.

11. Diblockcopolymer nach einem der vorhergehenden Ansprüche,
**dadurch gekennzeichnet,**
**dass** die Oberfläche des Diblockcopolymers durch kovalente Anbindung oberflächenmodifizierender Substanzen d) chemisch strukturiert ist, wobei als oberflächenmodifizierende Substanz d) Molekühle mit nukleophilen funktionellen Gruppen eingesetzt werden.

12. Diblockcopolymer nach einem der Ansprüche 1 bis 11,
**dadurch gekennzeichnet,**
**dass** das Diblockcopolymer zusätzlich mindestens eine oberflächenmodifizierende Substanz d) enthält, wobei die Substanz d) über die reaktive Gruppe c) mit dem hydrophilen Polymer b) verbunden ist.

13. Diblockcopolymer nach Anspruch 12,
**dadurch gekennzeichnet,**
**dass** die Substanz d) mindestens eine Substanz ist ausgewählt unter einem Kohlenhydrat, Peptid, Protein, Heteroglykan, Proteoglykan, Glykoprotein, Aminosäure, Fett, Phospholipid, Glykolipid, Lipoprotein, Arzneistoff, Antikörper, Enzym, DNA / RNA einer Zelle, Farbstoff und molekularen Sensor.

14. Formkörper gebildet aus einem Diblockcopolymer nach einem der Ansprüche 1 bis 13.

15. Formkörper nach Anspruch 14,
**dadurch gekennzeichnet,**
**dass** der Formkörper ein Film, Partikel, dreidimensionaler Körper, poröser Körper oder ein Schwamm ist.

16. Verwendung eines Diblockcopolymers nach einem der Ansprüche 1 bis 15 zur Herstellung von drug targeting-Systemen, drug delivery-Systemen, Bioreaktoren, für therapeutische und diagnostische Zwecke, für das tissue engineering und als Emulgator

17. Verfahren zur Herstellung eines Diblockcopolymers nach einem der Ansprüche 12 oder 13,
**dadurch gekennzeichnet,**
**dass** die mindesten eine Substanz d) mit einem Diblockcopolymer nach einem der Ansprüche 1 bis 11, wobei das Diblockcopolymer in Lösung oder in der Festphase vorliegt, umgesetzt wird.

18. Verfahren nach Anspruch 17,
**dadurch gekennzeichnet,**
**dass** für die Anbindung der mindestens einen Substanz d) das Diblockcopolymer nach einem der Ansprüche 1 bis 11 in Form eines porösen Formkörpers eingesetzt wird.

19. Verfahren zur Herstellung eines Diblockcopolymers nach einem der Ansprüche 12 oder 13 oder nach einem der Ansprüche 17 oder 18,
**dadurch gekennzeichnet,**
**dass** in einer ersten Stufe die Substanz d) mit einer reaktiven Gruppe c) versehen wird und in einer zweiten Stufe der Komplex aus Substanz d) und reaktiver Gruppe c) über die reaktive Gruppe c) an das hydrophile Polymer b) eines Diblockcopolymers aus einem hydrophoben Polymer a) und einem hydrophilen Polymer b) gebunden wird.

20. Verfahren zur Herstellung eines Diblockcopolymers nach einem der Ansprüche 12 oder 13 oder 17 bis 19,
**dadurch gekennzeichnet,**
**dass** die Anbindung der mindestens einen Substanz d) an die Oberfläche des Diblockcopolymers unter Erzeugung eines Substratmusters erfolgt.

21. Verfahren nach Anspruch 20,
**dadurch gekennzeichnet,**
**dass** die Substanz d) mit örtlich konstanter oder variabler Dichte über die reaktive Gruppe c) auf der Oberfläche eines Diblockcopolymers enthaltend eine hydrophobe Komponente a) und hydrophile Komponente b) aufgebracht wird.

22. Verfahren nach Anspruch 20 oder 21,
**dadurch gekennzeichnet,**
**dass** für die Aufbringung der reaktiven Gruppe c) und/oder der Substanz d) in einem Substratmuster die Oberfläche des Diblockcopolymers durch einen Plotter, einen Tintenstrahldrucker, Bestrahlung mit Licht, Beschuss mit Teilchen, Stempeln oder Soft Lithographie strukturiert wird.

## Claims

1. A diblock copolymer containing
a hydrophobic biodegradable polymer a),
a hydrophilic polymer b),
at least one reactive group c) for covalent binding of a surface-modifying substance d) to the hydrophilic polymer b), wherein the surface-modifying substance d) is a molecule with a nucleophilic functional group and is covalently bound by the reactive group c) without activation in aqueous medium, and wherein the at least one reactive group c) is selected from 1) a functional group and/or 2) an at least bifunctional molecule with at least one free functional group, wherein the reactive group c) has the ability to covalently bind the surface-modifying substance d) without activation in aqueous medium, provided that if the hydrophilic polymer b) is polyethylene glycol, the reactive group c) is not hydroxyl.

2. A diblock copolymer according to Claim 1,
**characterised in that**
the hydrophobic polymer a) and/or hydrophilic polymer b) are selected from a linear and/or branched polymer.

3. A diblock copolymer according to one of the preceding claims,
**characterised in that**
the hydrophobic polymer a) is at least one polymer selected from polyester, poly-ε-caprolactam, poly-α-hydroxyester, poly-β-hydroxyester, polyamide, polyphosphazene, polyanhydride, polydioxanon, polymalic acid, polytartaric acid, polyorthoester, polycarbonate, peptide, polysaccharide and protein.

4. A diblock copolymer according to Claim 3,
**characterised in that**
the hydrophobic polymer a) is at least one polymer selected from polylactide, polyglycolide, poly(lactide-co-glycolide), poly-β-hydroxybutyrate and poly-β-hydroxyvalerate.

5. A diblock copolymer according to one of the preceding claims, **characterised in that**
the hydrophilic polymer b) is at least one polymer selected from polyethylene glycol, polypropylene glycol, polyethylene glycol/polypropylene glycol copolymer, polyethylene glycol/polypropylene glycol/polyethylene glycol copolymer, polybutylene glycol, polyacrylamide, polyvinyl alcohol, polysaccharide, peptide and protein.

6. A diblock copolymer according to one of the preceding claims,
**characterised in that**
the reactive group c) is at least one selected from a keto group, an acid chloride, dicarboxylic acid amide, 3-maleic imidopropionic acid-N-succinimidyl ester and succinimidyl ester.

7. A diblock copolymer according to one of the preceding claims,
**characterised in that**
the hydrophobic polymer a) is at least one selected from polylactide, polyglycolide and poly(lactide-co-glycolide).

8. A diblock copolymer according to Claim 7,
**characterised in that**
the hydrophilic polymer b) is polyethylene glycol.

9. A diblock copolymer according to Claim 8,
**characterised in that**
the polyethylene glycol has a molar mass in a range of 200 to 10 000 Da.

10. A diblock copolymer according to one of the preceding claims,
**characterised in that**
the hydrophobic polymer a) is polylactide preferably with a molar mass in a range of 1 000 to 100 000 Da.

11. A diblock copolymer according to one of the preceding claims,
**characterised in that**
the surface of the diblock copolymer is chemically structured by covalent binding of surface-modifying substances d), wherein as surface-modifying substance d) there are used molecules with nucleophilic functional groups.

12. A diblock copolymer according to one of Claims 1 to 11,
**characterised in that**
the diblock copolymer additionally contains at least one surface-modifying substance d), wherein substance d) is bonded to the hydrophilic polymer b) by means of the reactive group c).

13. A diblock copolymer according to Claim 12,
**characterised in that**
the substance d) is at least one substance selected from a carbohydrate, peptide, protein, heteroglycan, proteoglycan, glycoprotein, amino acid, fat, phospholipid, glycolipid, lipoprotein, medicinal agent, antibody, enzyme, DNA/RNA of a cell, dye and molecular sensor.

14. A shaped body formed from a diblock copolymer according to one of Claims 1 to 13.

15. A shaped body according to Claim 14,
**characterised in that**
the shaped body is a film, particle, three-dimensional body, porous body or a sponge.

16. Use of a diblock copolymer according to one of Claims 1 to 15
for the production of drug-targeting systems, drug-delivery systems, bioreactors, for therapeutic and diagnostic purposes, for tissue engineering and as emulsifier.

17. A process for the production of a diblock copolymer according to one of Claims 12 or 13,
**characterised in that**
the at least one substance d) is converted with a diblock copolymer according to one of Claims 1 to 11, wherein the diblock copolymer is present in solution or in the solid phase.

18. A process according to Claim 17,
**characterised in that**
for the binding of the at least one substance d), the diblock copolymer according to one of Claims 1 to 11 is used in the form of a porous shaped body.

19. A process for the production of a diblock copolymer according to one of Claims 12 or 13 or according to one of Claims 17 or 18,
**characterised in that**
in a first stage, the substance d) is provided with a reactive group c) and in a second stage, the complex composed of substance d) and reactive group c) is bound by means of the reactive group c) to the hydrophilic polymer b) of a diblock copolymer composed of a hydrophobic polymer a) and a hydrophilic polymer b).

20. A process for the production of a diblock copolymer according to one of Claims 12 or 13 or according to one of Claims 17 to 19,
**characterised in that** the binding of the at least one substance d) to the surface of the diblock copolymer is achieved by generating a substrate pattern.

21. A process according to Claim 20,
**characterised in that**
the substance d) is applied with a locally constant or variable concentration by means of the reactive group c) on the surface of a diblock copolymer containing a hydrophobic component a) and hydrophilic component b).

22. A process according to Claim 20 or 21,
**characterised in that**
for the binding of the reactive group c) and/or the substance d) in a substrate pattern, the surface of the diblock copolymer is structured by a plotter, an ink jet printer, radiation with light, bombardment with particles, stamping or soft lithography.

## Revendications

1. Copolymère dibloc comprenant
un polymère biodégradable hydrophobe a),
un polymère hydrophile b),
au moins un groupe réactif c) pour la liaison covalente d'une substance modifiée en surface d) au polymère hydrophile b), sachant que la substance modifiée en surface d) est une molécule ayant un groupe fonctionnel nucléophile et est liée de manière covalente par le groupe réactif c), sans activation en milieu aqueux, et sachant que le au moins un groupe réactif c) est choisi parmi 1) un groupe fonctionnel et/ou 2) une molécule au moins bifonctionnelle ayant au moins un groupe fonctionnel libre, le groupe réactif c) ayant la capacité de lier de manière covalente la substance modifiée en surface d), sans activation en milieu aqueux, à la condition que, quand le polymère hydrophile b) est un polyéthylène glycol, le groupe réactif c) n'est pas un groupe hydroxyle.

2. Copolymère dibloc selon la revendication 1,
**caractérisé en ce que** le polymère hydrophobe a) et/ou le polymère hydrophile b) sont choisis parmi un polymère linéaire et/ou ramifié.

3. Copolymère dibloc selon l'une des revendications précédentes,
**caractérisé en ce que** le polymère hydrophobe a) est au moins un polymère choisi parmi un polyester, une poly-ε-caprolactone, un poly-α-hydroxyester, un poly-β-hydroxyester, un polyamide, les polyphosphases, un polyanhydride, une polydioxanone, un acide polymaléique, un acide polytartrique, un polyorthoester, un polycarbonate, un peptide, un polysaccharide et une protéine.

4. Copolymère dibloc selon la revendication 3,
**caractérisé en ce que** le polymère hydrophobe a) est au moins un polymère choisi parmi le polylactide, le polyglycolide, le poly(lactide-co-glycolide), le poly-β-hydroxybutyrate et le poly-β-hydroxyvalérate.

5. Copolymère dibloc selon l'une des revendications précédentes,
**caractérisé en ce que** le polymère hydrophile b) est au moins un polymère choisi parmi un polyéthylène glycol, un polypropylène glycol, un copolymère de polyéthylène glycol/polypropylène glycol, un copolymère de polyéthylène glycol/polypropylène glycol/polyéthylène glycol, un polybutylène glycol, un polyacrylamide, un alcool polyvinylique, un polysaccharide, un peptide et une protéine.

6. Copolymère dibloc selon l'une des revendications précédentes,
**caractérisé en ce que** le groupe réactif c) est au moins un groupe choisi parmi un groupe céto, un chlorure d'acide, un amide d'acide dicarboxylique, un ester de N-succinimidyle d'acide 3-maléinimidopropionique et un ester de succinimidyle.

7. Copolymère dibloc selon l'une des revendications précédentes,
**caractérisé en ce que** le polymère hydrophobe a) est au moins un polymère choisi parmi un polylactide, un polyglycolide et un poly(lactide-co-glycolide).

8. Copolymère dibloc selon la revendication 7,
**caractérisé en ce que** le polymère hydrophile b) est le polyéthylène glycol.

9. Copolymère dibloc selon la revendication 8,
**caractérisé en ce que** le polyéthylène glycol a une masse molaire allant de 200 à 10 000 Da.

10. Copolymère dibloc selon l'une des revendications précédentes,
**caractérisé en ce que** le polymère hydrophobe a) est un polylactide ayant de préférence une masse molaire allant de 1 000 à 100 000 Da.

11. Copolymère dibloc selon l'une des revendications précédentes,
**caractérisé en ce que** la surface du copolymère dibloc est structurée chimiquement par liaison covalente des substances modifiées en surface d), étant entendu que l'on utilise, comme substance modifiée en surface d), des molécules ayant des groupes fonctionnels nucléophiles.

12. Copolymère dibloc selon l'une des revendications 1 à 11,
**caractérisé en ce que** le copolymère dibloc comprend en plus au moins une substance modifiée en surface d), la substance d) étant liée au polymère hydrophile b) par le biais du groupe réactif c).

13. Copolymère dibloc selon la revendication 12,
**caractérisé en ce que** la substance d) est au moins une substance choisie parmi un hydrate de carbone, un peptide, une protéine, un hétéroglycane, un protéoglycane, une glycoprotéine, un acide aminé, une graisse, un phospholipide, un glycolipide, une lipoprotéine, un médicament, un anticorps, une enzyme, un ADN/ARN de cellule, un colorant et un capteur moléculaire.

14. Corps moulé formé en un copolymère dibloc selon l'une des revendications 1 à 13.

15. Corps moulé selon la revendication 14,
**caractérisé en ce que** le corps moulé est un film, une particule, un corps tridimensionnel, un corps poreux ou une éponge.

16. Utilisation d'un copolymère dibloc selon l'une des revendications 1 à 15 pour la préparation de systèmes de ciblage de médicament, de systèmes de distribution de médicaments, de bioréacteurs, à des fins thérapeutiques et diagnostiques, pour la manipulation des tissus et comme émulsifiant.

17. Procédé de préparation d'un copolymère dibloc selon l'une des revendications 12 ou 13,
**caractérisé en ce que** l'on fait réagir la moins une substance d) avec un copolymère dibloc selon l'une des revendications 1 à 11, le copolymère dibloc se présentant en solution ou en phase solide.

18. Procédé selon la revendication 17,
**caractérisé en ce que**, pour la liaison d'au moins la substance d), le copolymère dibloc selon l'une des revendications 1 à 11 est utilisé sous la forme d'un corps moulé poreux.

19. Procédé de préparation d'un copolymère dibloc selon l'une des revendications 12 ou 13 ou selon l'une des revendications 17 ou 18,
**caractérisé en ce que**, dans une première étape, la substance d) est dotée d'un groupe réactif c) et dans une deuxième étape, le complexe constitué de la substance d) et du groupe réactif c) est lié au polymère hydrophile b) d'un copolymère dibloc, constitué d'un polymère hydrophobe a) et d'un polymère hydrophile b), par le biais du groupe réactif c).

20. Procédé de préparation d'un copolymère dibloc selon l'une des revendications 12 ou 13 ou 17 à 19,
**caractérisé en ce que** la liaison d'au moins une substance d) à la surface d'un copolymère dibloc se fait en générant un modèle de substrat.

21. Procédé selon la revendication 20,
**caractérisé en ce que** la substance d) est déposée à un densité constante ou variable localement, par le biais du groupe réactif c), sur la surface d'un copolymère dibloc comprenant un composant hydrophobe a) et un composant hydrophile b).

22. Procédé selon la revendication 20 ou 21,
**caractérisé en ce que**, pour le dépôt du groupe réactif c) et/ou de la substance d) en un modèle de substrat, la surface du copolymère dibloc est structurée par un traceur, une imprimante à jets d'encre, une irradiation avec de la lumière, une projection de particules, un estampage ou la lithographie douce.
